# EUROPEAN PATENT APPLICATION

(11) **EP 4 806 416 A1**
(43) Date of publication of application: **16.09.2026**
(21) Application number: 24888598.0
(22) Date of filing: 29.10.2024
(51) Int. Cl.: A61K 8/19, A61K 8/02, A61K 8/25, A61Q 1/00, A61Q 1/04, A61Q 1/06, A61Q 1/08, A61Q 1/10, A61Q 3/02, A61Q 15/00, A61Q 19/00, C01B 33/42, C09C 3/06

(54) **SILICA-COATED PLATE-LIKE POWDER AND COSMETIC**

(30) Priority: 10.11.2023 JP 2023192356; 11.06.2024 JP 2024094460
(71) Applicant: SHIN-ETSU CHEMICAL CO., LTD., Tokyo 100-0005 (JP)
(72) Inventor: TANAKA Mitsuru, Tokyo 100-0005 (JP); TAKAGI Kazunori, Annaka-shi, Gunma 379-0224 (JP)
(74) Representative: Mewburn Ellis LLP
(86) International application number: PCT/JP2024/038503
(87) International publication number: WO 2025/100301

(57) **Abstract**

The present invention provides a composite powder and a cosmetic which have good usability at the time of application, attain a natural finish with transparency and suppressed gloss while correcting unevenness or the like of the skin, and impart an excellent effect in sustaining a cosmetic coating film.

## Description

### TECHNICAL FIELD

The present invention relates to a silica-coated plate-like powder that is coated with a layer of mesoporous silica, and to a cosmetic containing the same.

### BACKGROUND ART

One of the purposes of cosmetics is to make the skin look smooth and beautiful by concealing morphological problems, such as wrinkles, pores, and rough texture of the skin, as well as discoloration problems, such as blemishes and freckles on the skin. In recent years, emphasis has been placed on a natural finish that is not artificial (a bare skin feel). The cosmetic finish is natural when the cosmetic film is free of unnatural gloss (luster), is uniformly attached to the skin, and has high transparency. Many new materials and new technologies have been proposed so far to achieve a natural finish while still maintaining the above-mentioned effects of make-up cosmetics. In particular, cosmetics containing various types of diffuse reflective powders are known as a tool for concealing morphological problems.

The shapes of powders used in cosmetics are broadly divided into spherical shapes, plate-like shapes, and irregular shapes. Spherical powders mainly provide slipperiness and soft focus effects, while plate-like powders give smoothness. Many of plate-like powders have a glossy appearance. Those powders that are less glossy have a higher hiding ability. Irregular particles that vary in particle shape are difficult to apply uniformly, are unpleasant to the touch, and give an unnatural finish.

On the other hand, substances with a porous structure on the powder surface have a large surface area and are therefore widely used as catalyst carriers and carriers for immobilizing, for example, enzymes and functional organic compounds. Powders with a porous structure have been also used in cosmetics for the purpose of, for example, absorbing sebum from the skin and thereby preventing the cosmetic from coming off.

Attention has been drawn to the use of mesoporous powders with meso-sized pores that have been developed as porous bodies having uniform and fine pores. Patent Document 1 describes a mesoporous powder that effectively helps a cosmetic last long. However, the powder has an irregular shape and the addition thereof to a cosmetic can result in, for example, nonuniform dispersion, poor attachment to the skin upon application, and squeaky feeling after application. Patent Document 2 discloses that a mesoporous silica can be formed into a layer or a sheet. Unfortunately, the disclosed product gives a squeaky feeling when added to cosmetics, and thus needs improvements in the feeling on use. While the disclosure of Patent Documents 3 and 4 includes truly spherical particles with a mesoporous structure, and a hollow composite powder, there is no report on usability.

### PRIOR ART DOCUMENTS

### PATENT DOCUMENTS

Patent Document 1: JP-A H10-152317
Patent Document 2: JP-A 2008-266049
Patent Document 3: JP-A 2008-063209
Patent Document 4: JP-A 2008-174435

### SUMMARY OF INVENTION

### TECHNICAL PROBLEM

The present invention has been made in view of the circumstances discussed above. Objects of the present invention are therefore to provide a composite powder that shows good usability at the time of application, gives a natural finish with transparency and moderate luster while correcting problems, such as unevenness on the skin, and highly effectively helps a cosmetic film last long, and to provide a related cosmetic.

### SOLUTION TO PROBLEM

The present inventors carried out extensive studies directed to solving the problems described above. As a result, the present inventors have found that a composite powder in which a plate-like powder is coated with a layer of mesoporous silica is useful in cosmetics, and allows a cosmetic to show good usability at the time of application, give a natural finish with transparency and moderate luster while correcting problems, such as unevenness on the skin, and effectively form a long-lasting cosmetic film. The present invention has been completed based on the finding.

Specifically, the present invention provides the following silica-coated plate-like powders and cosmetics.
1. A silica-coated plate-like powder comprising a plate-like powder and a coating layer of mesoporous silica.
2. The silica-coated plate-like powder according to 1, wherein the mesoporous silica represents 10 to 60% by mass of the silica-coated plate-like powder taken as 100% by mass.
3. The silica-coated plate-like powder according to 1, wherein spherical silica is further immobilized.
4. The silica-coated plate-like powder according to 3, wherein the spherical silica has an average particle diameter of 100 to 500 nm.
5. The silica-coated plate-like powder according to 3 or 4, wherein the amount of the spherical silica is 5 to 25% by mass relative to the silica-coated plate-like powder taken as 100% by mass.
6. The silica-coated plate-like powder according to any one of 1 to 5, which has a BET specific surface area of 10 to 400 m²/g.
7. The silica-coated plate-like powder according to any one of 1 to 6, wherein the plate-like powder is a mineral, a metal oxide, a metal, or glass.
8. The silica-coated plate-like powder according to any one of 1 to 6, wherein the plate-like powder is mica.
9. A cosmetic including the silica-coated plate-like powder described in any one of 1 to 8.
10. The cosmetic according to 9, which is a skin care cosmetic.
11. The cosmetic according to 9, which is a make-up cosmetic.
12. A silica-coated plate-like powder coated with a layer of silica having pores, the silica-coated plate-like powder being obtained by steps including:
   step (1): preparing an aqueous solution containing a cationic surfactant at a concentration 5 times or less a critical micelle concentration;
   step (2): immersing a plate-like powder in the aqueous solution obtained in the step (1), adding a silica source that generates a silanol compound upon hydrolysis to a concentration of 10 to 500 mmol/L, and stirring the mixture at a temperature of 0 to 100°C to coat a surface of the plate-like powder with a layer of mesoporous silica; and
   step (3): firing the resultant silica-coated plate-like powder coated with a mesoporous silica layer to remove the cationic surfactant.
13. A method for producing a silica-coated plate-like powder coated with a layer of silica having pores, the method including:
   step (1): preparing an aqueous solution containing a cationic surfactant at a concentration 5 times or less a critical micelle concentration;
   step (2): immersing a plate-like powder in the aqueous solution obtained in the step (1), adding a silica source that generates a silanol compound upon hydrolysis to a concentration of 10 to 500 mmol/L, and stirring the mixture at a temperature of 0 to 100°C to coat a surface of the plate-like powder with a layer of mesoporous silica; and
   step (3): firing the resultant silica-coated plate-like powder coated with a mesoporous silica layer to remove the cationic surfactant.

### ADVANTAGEOUS EFFECTS OF INVENTION

The silica-coated plate-like powder of the present invention allows a cosmetic to show good usability at the time of application, give a natural finish with transparency and moderate luster while correcting problems, such as unevenness on the skin, and effectively form a long-lasting cosmetic film.

### DESCRIPTION OF EMBODIMENTS

The present invention will be described in detail below. In the present invention, the name of ingredients is sometimes expressed by the Japanese labeling name or the International Nomenclature of Cosmetic Ingredients (INCI). When the Japanese labeling name is identical with the INCI, sometimes either one is omitted. The present invention pertains to a silica-coated plate-like powder that is coated with a layer of mesoporous silica. The powder of the present invention is particularly useful in cosmetics, but the use thereof is not limited thereto.

### [Plate-like powders]

A plate-like powder to be coated will be described. The term "plate-like powder" indicates powder particles having a plate-like shape. When used in cosmetics, such a powder exhibits excellent smoothness and adhesion to the skin after the cosmetic is applied. The plate-like powder has a flat surface. Thus, the plate-like powder strongly reflects light incident on the surface of the cosmetic film. Since the plate-like powder reflects light in one direction without diffusing the light, the reflected light is intense. In this manner, the plate-like powder, when applied to a cosmetic, effectively produces an enhanced luster. In the present invention, the plate-like powder is coated with a layer of mesoporous silica to give a sense of transparency with moderate luster.

The thickness of the plate-like powder of the present invention is defined by the aspect ratio [(major axis diameter of particle)/(minor axis diameter of particle)]. The thickness of the plate-like powder in the present invention is not particularly limited, but is preferably 3 to 500, and more preferably 10 to 300. The aspect ratio may be measured by observation with a scanning electron microscope or a transmission electron microscope. The size of the plate-like powder is not particularly limited, but the average particle diameter is preferably 1 to 100 µm, more preferably 1 to 50 µm, and still more preferably 3 to 40 µm. The average particle diameter is a value of volume median diameter (D50). The volume median diameter (D50) is the particle diameter equivalent to 50% cumulative volume in the particle size distribution. For example, the volume median diameter is a value measured with a laser diffraction/scattering particle size distribution analyzer.

The plate-like powder is not particularly limited as long as the powder is generally used in cosmetics. Examples include minerals, metal oxides, metals, and glass. The plate-like powders may be used singly, or two or more may be used in combination.

Examples of the minerals include talc, mica, and kaolin. Examples of the metal oxides include silica. Examples of the metals include gold and silver. Examples of the glass include (calcium/aluminum) borosilicate. Other plate-like powders that can be added to cosmetics include barium sulfate and boron nitride. Micas are preferable because they have a refractive index close to that of the skin, and show transparency and appropriate gloss when applied to the skin. Any micas generally used in cosmetics may be used without limitation. Specifically, suitable micas include mica series (manufactured by YAMAGUCHI MICA CO., LTD.), such as TM-10, TM-20, Y-1800, Y-2300, Y-2300X, Y-2400, Y-3000, SA-110, SA-310, SA-350, FA-450, NCC-322, and NCF-322; RONAFLAIR series (manufactured by Merck), such as RONAFLAIR Mica M and RONAFLAIR Silk Mica; Mearlmica series (manufactured by Merck), such as Mearlmica CF and Mearlmica SV; Sericite Powder series (manufactured by JAPAN SERICITE CORPORATION), such as FSE, FSE-S, FSN, NSP, JS-A, and JS-1; Eight Pearl series (manufactured by Kakuhachi Co., Ltd.), such as Eight Pearl 300S, Eight Pearl 300SW, and Eight Pearl 300S-S; Sericite DN-MC (manufactured by Dainihonkasei Co., Ltd.); and GMS series (manufactured by KINSEI MATEC CO., LTD.), such as Sericite GMS-C and Sericite GMS-4C.

### [Silica-coated plate-like powders coated with a layer of mesoporous silica]

In the present invention, the plate-like powder is coated with a layer of mesoporous silica. The phrase "coated with a layer" means that the silica does not take a particulate shape on the powder surface. Such silica effectively lowers the brightness of the plate-like powder.

In the present invention, the silica layer covering the plate-like powder has mesoporous pores. In the present invention, mesoporous pores refer to pores with a diameter of 50 nm or less. The diameter of the pores can be selected appropriately and may be less than 2 nm, may be 0.5 nm or more and less than 2 nm, may be 2 to 50 nm, or may be 2 to 10 nm. The lower limit is not particularly limited and may be 0.1 nm. As compared to a general silica coating layer, the mesoporous silica layer has fine pores and a large specific surface area, and therefore effectively scatters light and absorbs sebum to allow a cosmetic film to remain effective longer. The diameter of the pores is measured by the BJH method using a high-precision gas adsorption analyzer. For example, the diameter of the pores may be controlled appropriately by selecting the temperature and the amount of time in step (3) or step (3') described later.

### [Methods for producing the silica-coated plate-like powders]

A method for producing the silica-coated plate-like powder in the present invention will be described in detail below. The production method is not limited to the following.

The method for producing the silica-coated plate-like powder of the present invention includes the following steps (1) to (3):
step (1): preparing an aqueous solution containing a cationic surfactant at a concentration 5 times or less the critical micelle concentration;
step (2): immersing a plate-like powder in the aqueous solution obtained in the step (1), adding a silica source that generates a silanol compound upon hydrolysis to a concentration of 10 to 500 mmol/L, and stirring the mixture at a temperature of 0 to 100°C to coat the surface of the plate-like powder with a layer of mesoporous silica; and
step (3): removing the cationic surfactant from the silica-coated plate-like powder coated with a mesoporous silica layer.

The steps and the components used in the steps will be described below.

### [Step (1)]

In the step (1), an aqueous solution of a cationic surfactant is prepared. In the production method, mesoporous silica is formed using this aqueous solution. The cationic surfactant is used to form a mesoporous structure and to disperse silica particles and a plate-like powder. The cationic surfactant used here may be a known one. The cationic surfactant is preferably an alkyltrimethylammonium salt represented by the following formula (1):

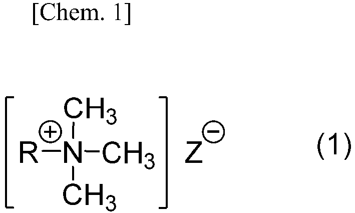

### (In the formula, R is a C1-C22 alkyl group and Z is a monovalent anion.)

R is a C1-C22 alkyl group. The number of carbon atoms is preferably 8 to 15, and more preferably 10 to 13. The alkyl group may be linear, branched, or cyclic. Specific examples thereof include methyl, ethyl, n-propyl, isopropyl, n-butyl, isobutyl, s-butyl, t-butyl, n-pentyl, n-hexyl, cyclohexyl, n-heptyl, n-octyl, n-nonyl, n-decyl, n-undecyl, n-dodecyl, 2-methylundecyl, n-tridecyl, n-tetradecyl, n-pentadecyl, n-hexadecyl, n-heptadecyl, and n-octadecyl.

Z is a monovalent anion, preferably a halide ion, a hydroxide ion, a nitrate ion, or a sulfate ion, more preferably a halide ion, and still more preferably a chloride ion or a bromide ion.

The cationic surfactant is preferably an alkyltrimethylammonium salt. Specific examples thereof include butyltrimethyl ammonium chloride, hexyltrimethylammonium chloride, octyltrimethylammonium chloride, decyltrimethylammonium chloride, dodecyltrimethylammonium chloride, tetradecyltrimethylammonium chloride, hexadecyltrimethylammonium chloride, stearyltrimethylammonium chloride, butyltrimethylammonium bromide, hexyltrimethylammonium bromide, octyltrimethylammonium bromide, decyltrimethylammonium bromide, dodecyltrimethylammonium bromide, tetradecyltrimethylammonium bromide, hexadecyltrimethylammonium bromide, and stearyltrimethylammonium bromide. Among these, dodecyltrimethylammonium chloride is preferable from the point of view of forming a uniform mesoporous structure.

From the point of view of powder dispersibility, the concentration of the cationic surfactant in the aqueous solution in the step (1) is preferably 5 times or less, more preferably 4 times or less, and still more preferably 3 times or less the critical micelle concentration of the cationic surfactant that is used, in water at 20°C.

Step (2): A plate-like powder is immersed in the aqueous solution obtained in the step (1), a silica source that generates a silanol compound upon hydrolysis is added to a concentration of 10 to 500 mmol/L, and the mixture is stirred at a temperature of 0 to 100°C to coat the surface of the plate-like powder with a layer of mesoporous silica.

The concentration of the silica source is 10 to 500 mmol/L, preferably 50 to 250 mmol/L, and more preferably 75 to 200 mmol/L. The temperature is 0 to 100°C, and more preferably 5 to 80°C. The amount of time for which the solution is stirred in the step (2) varies depending on temperature, but is usually selected appropriately from 0.1 to 24 hours. The silica source is not particularly limited as long as it is a compound that generates a silanol compound upon hydrolysis. Examples thereof include tetramethyl orthosilicate, tetraethyl orthosilicate, and tetraisopropyl orthosilicate. In particular, tetramethyl orthosilicate, such as tetramethoxysilane, is preferable.

Step (3): The cationic surfactant is removed from the silica-coated plate-like powder coated with a mesoporous silica layer.

In the step (3), the plate-like powder coated with a mesoporous silica layer, obtained in the step (2), is taken out of the aqueous solution and is washed with water and dried as required, and subsequently the cationic surfactant is removed from the plate-like powder. For example, the cationic surfactant may be removed by extraction or firing. In extraction, the cationic surfactant may be extracted by adding the mesoporous silica layer-coated substrate into an aqueous solution having a pH of 1 to 4 and a temperature of 25 to 80°C, and stirring the mixture for 1 to 48 hours, preferably 2 to 24 hours, and more preferably 3 to 16 hours. It is, however, difficult to completely remove the cationic surfactant by extraction. In the case of firing, the cationic surfactant can be removed by firing the powder in an electric furnace or the like, preferably at 400 to 700°C, more preferably at 500 to 650°C, for 1 to 10 hours.

The proportion of the mesoporous silica is preferably 10 to 60% by mass, and more preferably 20 to 40% by mass, relative to the silica-coated plate-like powder taken as 100% by mass. When the proportion is 10% by mass or more, the silica layer covering the powder produces its effects. When the proportion is 60% by mass or less, the thickness of the layer is appropriate, and the particles do not aggregate to one another and are therefore easy to disperse and apply uniformly, thus realizing enhanced usability.

### [Immobilized spherical silica-bearing silica-coated composite powders]

The silica-coated plate-like powder of the present invention preferably further has spherical silica immobilized on the surface thereof. By the immobilization of spherical silica, further enhancements are obtained in usability and in light scattering effects that correct problems, such as unevenness on the skin, and also high slipperiness is imparted. The spherical silica is immobilized and is resistant to separation from the surface of the silica coating. It is preferable that the immobilized spherical silica-bearing silica-coated composite powder have a plate-like shape.

### [Spherical silica]

The spherical silica of the present invention will be described. When used in a cosmetic, spherical silica particles act as a kind of ball bearing during the application of the cosmetic and dramatically enhances the slipperiness of the composite powder in an effective manner. Furthermore, spherical silica particles effectively scatter light on the surface of the cosmetic film and thereby effectively hide morphological problems on the skin related to light reflection (produce soft focus effects).

In the spherical silica of the present invention, the term "spherical" includes not only perfect spheres, but also slightly distorted spheres. The shape of such particles may be evaluated as the circularity of the particles that are projected two-dimensionally. To determine the circularity in the present invention, the particles are photographed with a scanning electron microscope (SEM), and the major axis and the minor axis of randomly selected 30 particles are measured to calculate the minor axis/major axis ratio of each particle, and the minor axis/major axis ratios are averaged. Here, the term "spherical" in the present invention means, in particular, that the circularity is in the range of 0.8 to 1. The circularity is preferably 0.85 to 1, and more preferably 0.90 to 1.

For example, the average particle diameter of the spherical silica may be 80 to 550 nm, and is preferably 100 to 500 nm, and more preferably 200 to 500 nm. Here, the average particle diameter is a value of volume median diameter (D50). The volume median diameter (D50) is the particle diameter equivalent to 50% cumulative volume in the particle size distribution. For example, the volume median diameter is a value measured with a laser diffraction/scattering particle size distribution analyzer. The above range ensures that the immobilized spherical silica will produce light scattering effects and will correct skin unevenness effectively. In addition, the spherical silica falling in the above range has a small area of contact with the skin and advantageously offers excellent slipperiness.

The spherical silica used in the present invention may be hydrophobic spherical silica obtained by hydrophobic treatment on the surface. Hydrophilic spherical silica may be used as such without surface treatment. When the powder will be dispersed in an aqueous component, hydrophilic spherical silica is preferable. When the powder will be dispersed in an oily component, hydrophobic spherical silica is preferable.

The spherical silica in the present invention may be non-porous or porous, and may be microporous, mesoporous, or microporous. Non-porous spherical silica exhibits good slipperiness on the skin and offers good spreadability. Porous spherical silica is expected to provide soft focus effects due to its larger specific surface area.

Suitable silicas include silica, hydrated silica, silica dimethicone silylate, and silica dimethyl silylate, defined in the Japanese labeling name.

The spherical silica in the present invention may be produced by any known method without limitation, such as, for example, sol-gel method. In the sol-gel method, a tetrafunctional silane compound is added to a mixture of a hydrophilic organic solvent, such as alcohol, water, and a basic substance, the resultant mixture is reacted to give a solvent dispersion containing hydrophilic spherical silica microparticles, and the solvent is dried to leave the powder.

It is preferable that the spherical silica be chemically immobilized to the surface of the silica-coated plate-like powder. The spherical silica may be immobilized to the surface in any manner without limitation but is preferably immobilized through a binder. That is, the spherical silica may be immobilized to the surface of the silica-coated plate-like powder that has been coated with a binder layer. Such a composite powder is advantageous in that the spherical silica is firmly immobilized to the powder surface and is resistant to separation from the surface, thus giving a better feeling on use. As a result of chemical immobilization, the spherical silica that has been firmly immobilized to the substrate is prevented from separation due to stress caused by mechanical factors during the production of a cosmetic, and the powder can produce effects even after the cosmetic manufacturing process.

Examples of the binders include general silane coupling agents, such as alkoxysilanes, and partial hydrolysates thereof. When a partial hydrolysate of a silane coupling agent is used as the binder, the state of adhesion to the surface of the plate-like powder may be as a film or particles. Such a binder may be attached to part or the whole of the surface of the plate-like powder, the surface of the spherical silica, or the surface of both. In particular, it is preferable that the binder be attached as a film to the entire interface between the silica-coated plate-like powder and the spherical silica.

### [Methods for immobilizing the spherical silica to the silica-coated plate-like powder]

The spherical silica may be immobilized to the silica-coated plate-like powder in the present invention by any method without limitation. An exemplary method will be described below.

The method for producing the immobilized spherical silica-bearing silica-coated composite powder includes the following steps (1) to (3'):
step (1): preparing an aqueous solution containing a cationic surfactant at a concentration 5 times or less the critical micelle concentration;
step (2'): immersing the silica-coated plate-like powder in the aqueous solution obtained in the step (1), adding spherical silica having a median diameter of 0.1 to 1 µm in an amount of 1 to 50% by mass relative to 100% by mass of the silica-coated plate-like powder, further adding a silica source that generates a silanol compound upon hydrolysis so that the concentration will be 10 to 500 mmol/L, and stirring the mixture at a temperature of 0 to 100°C to immobilize the spherical silica to the surface of the silica-coated plate-like powder; and
step (3'): removing the cationic surfactant from the immobilized spherical silica-bearing silica-coated composite powder.

### Step (1)

The step (1) is the same as the step (1) in the method for producing the silica-coated plate-like powder described above.

Step (2'): The silica-coated plate-like powder is immersed in the aqueous solution obtained in the step (1); spherical silica having a median diameter of 0.1 to 1 µm is added in an amount of 1 to 50% by mass relative to 100% by mass of the silica-coated plate-like powder; a silica source that generates a silanol compound upon hydrolysis is further added so that the concentration will be 10 to 500 mmol/L; and the mixture is stirred at a temperature of 0 to 100°C to immobilize the spherical silica to the surface of the silica-coated plate-like powder.

The amount in which the spherical silica is added (attached) is preferably 5 to 25% by mass, more preferably 7.5 to 20% by mass, and still more preferably 10 to 15% by mass, relative to 100% by mass of the silica-coated plate-like powder that is coated with a layer of mesoporous silica. The light scattering effects are further enhanced by controlling this amount to 5% by mass or more. When the amount is controlled to 25% by mass or less, the substrate can produce its effect and a more natural finish is obtained while benefitting from light scattering effects.

The concentration of the silica source is 10 to 500 mmol/L, preferably 50 to 250 mmol/L, and more preferably 75 to 200 mmol/L. The temperature is 0 to 100°C, and more preferably 5 to 80°C. The amount of time for which the solution is stirred in the step (2') varies depending on temperature, but is usually selected appropriately from 0.1 to 24 hours. The silica source may be similar to that described in the step (2) in the method for producing the silica-coated plate-like powder.

Step (3'): The cationic surfactant is removed from the immobilized spherical silica-bearing silica-coated composite powder.

In the step (3'), the immobilized spherical silica-bearing silica-coated composite powder, obtained in the step (2'), is taken out of the aqueous solution and is washed with water and dried as required, and subsequently the cationic surfactant is removed from the composite powder. For example, the cationic surfactant may be removed by extraction or firing. In extraction, the cationic surfactant may be extracted by adding the composite powder into an aqueous solution having a pH of 1 to 4 and a temperature of 25 to 80°C, and stirring the mixture for 1 to 48 hours, preferably 2 to 24 hours, and more preferably 3 to 16 hours. It is, however, difficult to completely remove the cationic surfactant by extraction. In the case of firing, the cationic surfactant can be removed by firing the powder in an electric furnace or the like, preferably at 400 to 700°C, more preferably at 500 to 650°C, for 1 to 10 hours.

### [Properties of the silica-coated plate-like powders]

The BET specific surface area of the silica-coated plate-like powder (or the immobilized spherical silica-bearing silica-coated composite powder) of the present invention is preferably 10 to 400 m²/g, and more preferably 20 to 200 m²/g. Controlling the specific surface area to 10 m²/g or more provides further enhancements in light scattering properties and oil absorption properties. On the other hand, controlling the specific surface area to 400 m²/g or less can reduce dryness while further enhancing light scattering properties and oil absorption properties.

The thickness of the silica-coated plate-like powder of the present invention is defined by the aspect ratio [(major axis diameter of particle)/(minor axis diameter of particle)]. The thickness of the silica-coated plate-like powder in the present invention is not particularly limited, but is preferably 3 to 500, and more preferably 10 to 300. The size of the silica-coated plate-like powder is not particularly limited, but the average particle diameter is preferably 1 to 100 µm, more preferably 1 to 50 µm, and still more preferably 3 to 40 µm.

The silica-coated plate-like powder is preferably composite particles in which mica is coated with a layer of mesoporous silica and spherical silica is chemically immobilized. In this case, the spherical silica on the surface provides slipperiness and skin unevenness correcting effects, the mica and the spherical silica effectively reduce the unnaturalness in luster, and the composite powder, when added to a foundation or the like, can give a natural finish with transparency and moderate luster. In addition to the effect of giving a natural finish, the composite powder, because of its having a large specific surface area, exhibits enhanced oil absorption properties and is expected to allow a cosmetic film to last long by adsorbing sebum. Furthermore, the chemical immobilization increases the strength of the composite powder, allowing the composite powder to withstand a mechanical stress expected in the cosmetic manufacturing process. Thus, the effects of the composite powder of the present invention are not impaired.

In order to impart or enhance water repellency or to improve the dispersibility in oils, the surface of the silica-coated plate-like powder of the present invention may be treated with, for example, a silylating agent, a silicone oil, a wax, a paraffin, an organic fluorine compound, and/or a surfactant. The treatment agent for hydrophobizing the surface (the hydrophobizing agent) is not particularly limited as long as the agent can impart hydrophobicity. Examples include silicone treatment agents, waxes, paraffins, organic fluorine compounds, such as perfluoroalkyl phosphate salts, surfactants, amino acids, such as N-acylglutamic acid, and metal soaps, such as aluminum stearate and magnesium myristate. The treatment agents may be used singly, or two or more may be used in combination.

In particular, silicone treatment agents are preferable, with examples including silanes or silylating agents, such as Triethoxycaprylylsilane (INCI) and Trimethoxysilyl Dimethicone (INCI), Dimethicone (INCI), Methicone (INCI), Hydrogen Dimethicone (INCI), Triethoxysilylethyl Polydimethylsiloxyethyl Dimethicone (INCI), Triethoxysilylethyl Polydimethylsiloxyethylhexyl Dimethicone (INCI), and (acrylates/tridecyl acrylate/triethoxysilylpropyl methacrylate/dimethicone methacrylate) copolymer (labeling name, INCI: Acrylates/Tridecyl Acrylate/Triethoxysilylpropyl Methacrylate/Dimethicone Methacrylate Copolymer). In particular, the silicone powder treatment agents described in Japanese Patent No. 3912961 are suitably used. Specific examples of the silicone treatment agents include AES-3083, KF-99P, KF-9901, KF-9908, KF-9909, KP-574, and KP-541, manufactured by Shin-Etsu Chemical Co., Ltd. In particular, for example, triethoxysilylethyl polydimethylsiloxyethylhexyl dimethicone (KF-9909, manufactured by Shin-Etsu Chemical Co., Ltd.), which is a dimethylpolysiloxane having a triethoxysilyl group, a polydimethylsiloxyethyl group, and a hexyl group in side chains, is preferable for the reason that it exhibits high affinity even when the dispersion medium for dispersing the powder is a silicone, a hydrocarbon, or a mixture thereof.

### [Surface treatment methods]

The silica-coated plate-like powder of the present invention may be surface-treated with the hydrophobizing agent by any known method without limitation. The surface treatment methods are largely classified into dry process and wet process. In a dry process, for example, the treatment may be performed by mixing/contacting the silica-coated plate-like powder of the present invention with the hydrophobizing agent using an appropriate stirring machine, crusher, mixer, disperser, or the like, such as a Henschel mixer, a ball mill, a jet mill, a kneader, a planetary mixer, a sand mill, an attritor, a ribbon blender, a dispersing mixer, or a homomixer. In this process, the treatment may be performed while applying energy, such as heat, mechanochemical mechanical force, or overheated steam. The treatment may also be performed in such a manner that the composite powder of the present invention and the hydrophobizing agent are sufficiently mixed/contacted and thereafter energy, such as heat, mechanochemical mechanical force, or overheated steam, is separately applied. In order to enhance the efficiency in dispersing the hydrophobizing agent in the process of mixing/contacting the hydrophobizing agent with the silica-coated plate-like powder of the present invention, for example, the hydrophobizing agent may be dissolved or dispersed beforehand in an appropriate amount of water, solvent, or supercritical fluid, and the resultant solution or dispersion may be sprayed to the silica-coated plate-like powder of the present invention. In a wet process, the treatment may be performed in such a manner that the silica-coated plate-like powder of the present invention and the hydrophobizing agent are dispersed in water, solvent, or supercritical fluid, thereby being mixed/contacted with each other, thereafter the solvent is evaporated, and further energy, such as heat, mechanochemical mechanical force, or overheated steam, is separately applied.

In order to make effective use of the silica-coated plate-like powder of the present invention, it is preferable that the silica-coated plate-like powder of the present invention be spread as a monolayer or in a similar state on the surface of the skin or on the surface of a cosmetic film. When, for example, the silica-coated plate-like powder of the present invention is used in a foundation or the like, it is preferable that the foundation be spread thinly and evenly. When the silica-coated plate-like powder of the present invention is used in a cosmetic for finishing or a cosmetic for fixing up the make-up, the advantageous effects of the present invention are more effectively produced by applying the cosmetic so as to form a thin film of the silica-coated plate-like powder.

### [Cosmetics containing the silica-coated plate-like powder]

The present invention is applicable to various types of cosmetics, and is particularly preferably applied to cosmetics externally applied to the skin, such as skin care cosmetics, make-up cosmetics, antiperspirant cosmetics, and sun care cosmetics, cosmetics externally applied to the hair, such as hair care cosmetics, and nail cosmetics. The amount of the silica-coated plate-like powder in the cosmetic is not limited, but is appropriately selected from the range of 0.1 to 99% by mass.

Exemplary skin care cosmetics include face lotions, milky lotions, creams, cleansing agents, packs, oil liquids, massage creams, cosmetic liquids, cosmetic oils, detergents, deodorants, hand creams, lip creams, and anti-wrinkle agents. Exemplary make-up cosmetics include make-up bases, foundations, concealers, cosmetic powders, cheek colors, eye colors, eyeshadows, mascaras, eye liners, eyebrows, and lip cosmetics. Exemplary antiperspirant cosmetics include antiperspirant cosmetics of roll-on type, cream type, solution type, and stick type. Exemplary sun care cosmetics include sunscreen oils, sunscreen milky lotions, and sunscreen creams. Exemplary hair care cosmetics include shampoos, conditioners, treatments, and setting agents.

The type of the cosmetic of the present invention may be any of, for example, powder, oil-based liquid, water-in-oil emulsion, oil-in-water emulsion, non-aqueous emulsion, or multi-emulsion, such as W/O/W or O/W/O emulsion. The form of the cosmetic of the present invention may be selected from various forms including liquid, milky lotion, cream, solid, paste, gel, powder, pressed, multilayer, mousse, spray, stick, and pencil.

The present invention has been made in consideration of the circumstances discussed hereinabove, and can provide a composite powder that shows good usability at the time of application, gives a natural finish with transparency and moderate luster while correcting problems, such as unevenness on the skin, and highly effectively helps a cosmetic film last long. Related cosmetics are also provided. More specifically, for example, emulsions excel in usability (spreadability and uniform attachment to the skin) at the time of application, can correct problems, such as unevenness on the skin (can conceal blemishes and wrinkles), and give a natural finish with transparency and moderate luster; sunscreen creams excel in usability (spreadability and uniform attachment to the skin) at the time of application, can correct problems, such as unevenness on the skin (can conceal blemishes and wrinkles), and give long-lasting cosmetic films (the films do not look oily); and powder foundations excel in usability (powder pick-up, spreadability, and uniform attachment to the skin) at the time of application, can correct problems, such as unevenness on the skin (can conceal blemishes and wrinkles), give a natural finish with transparency and moderate luster, and give long-lasting (persistent) cosmetic films. In view of the fact that powder foundations are highly loaded with powder, the present invention is further advantageous in that the powder is easy to mold and is not squeaking after application.

The cosmetic of the present invention may contain various components that are commonly used in cosmetics as long as the advantageous effects of the present invention are not impaired. Suitable components include, for example, (1) oils, (2) aqueous components, (3) surfactants, (4) powders other than the inventive powders, (5) compositions consisting of a crosslinked organopolysiloxane and an oil that is liquid at room temperature, (6) film-forming agents, (7) UV absorbing/scattering agents, and (8) other additives. These may be used singly, or two or more may be used in appropriate combination.

### (1) Oils

The oils may be volatile or nonvolatile and may be solid, semisolid, or liquid at room temperature (25°C). Examples include silicone oils, silicone waxes, natural animal and plant oils and fats and semi-synthetic oils and fats, hydrocarbon oils, higher alcohols, fatty acids, ester oils, fluorinated oils, and UV absorbers. These may be used singly, or two or more may be used in combination.

### • Silicone oils

Examples of the silicone oils include Dimethicone (INCI), Trisiloxane (INCI), alkyl-modified silicones, such as Methyl Trimethicone (INCI), Ethyl Trisiloxane (INCI), Ethyl Methicone (INCI), and Hexyl Dimethicone (INCI), long chain alkyl-modified silicones, such as Caprylyl Methicone (INCI), low to high viscosity linear or branched organopolysiloxanes, such as Phenyl Trimethicone (INCI), Diphenyl Dimethicone (INCI), Diphenylsiloxyphenyl Trimethicone (INCI), Tetraphenyl Dimethyldisiloxane (INCI), and methyl hydrogen polysiloxane, cyclic organopolysiloxanes, such as Cyclotetrasiloxane (INCI), Cyclopentasiloxane (INCI), and Cyclohexasiloxane (INCI), amino-modified organopolysiloxanes, such as Amodimethicone (INCI) and Aminopropyl Dimethicone (INCI), pyrrolidone-modified organopolysiloxanes, such as PCA Dimethicone (INCI), pyrrolidone carboxylic acid-modified organopolysiloxanes, silicone rubbers, such as gum-like dimethylpolysiloxane having a high degree of polymerization, gum-like amino-modified organopolysiloxane, and gum-like dimethylsiloxane-methylphenylsiloxane copolymers, as well as low viscosity organopolysiloxane solutions of silicone gums and rubbers, amino acid-modified silicones, fluorine-modified silicones, silicone resins, and silicone resin solutions.

Commercially available examples of the silicone oils include KF-96L-1cs, KF-96L-1.5cs, KF-96L-2cs, KF-96A-6cs, TMF-1.5, KF-4422, KF-4418, KF-54, KF-54HV, KF-56A, and KF-995, manufactured by Shin-Etsu Chemical Co., Ltd.

### • Solid oily components

When it is desired that the cosmetic of the present invention be solidified, an oily component that is solid at 25°C is preferably blended. Examples of the oily components that are solid at 25°C include waxes, hydrocarbons, esters, higher alcohols, and higher fatty acids having a melting point of preferably at least 40°C, more preferably 60 to 110°C. The oily component is not particularly limited as long as it is commonly blended in cosmetics. Specific examples include plant waxes, such as carnauba wax (INCI: Copernicia Cefifera (Carnauba) Wax), sugar cane wax, candelilla wax (INCI: Euphorbia Cerifera (Candelilla) Wax), purified candelilla wax, rice wax, Japan wax, jojoba wax, kapok wax, rice bran wax, myrica cerifera fruit wax, shea butter, cacao butter, Japan wax (INCI: Rhus Succedanea Fruit Wax), montan wax (INCI: Montan Wax), and hydrogenated castor oil isostearate; animal waxes, such as beeswax, beef tallow, beef bone fat, lard (INCI: Lard), horse fat (INCI: Horse Fat), mutton tallow, lanolin (INCI: Lanolin), insect wax, shellac wax, and sperm wax; semi-synthetic waxes, such as lanolin esters, lanolin fatty acid esters, and beeswax acid esters; hydrogenated oils, such as hydrogenated castor oil and hydrogenated coconut oil; hydrocarbon waxes, such as solid paraffin, polyethylene, ceresin, ozokerite, and microcrystalline wax; wax esters, such as synthetic beeswax; dioctyldodecyl lauroylglutamate, fatty acids, such as stearic acid and behenic acid; silicone waxes, such as acrylate silicone resins in the form of acrylate silicone graft or block copolymers (such as acrylate silicone graft copolymers: KP-561P and 562P, manufactured by Shin-Etsu Chemical Co., Ltd.); and derivatives thereof.

### • Natural animal and plant oils and semi-synthetic oils

Examples of the natural animal and plant oils and the semi-synthetic oils include naturally occurring plant oils, such as avocado oil (labeling name, INCI: Persea Gratissima (Avocado) Oil), linseed oil (labeling name, INCI: Linum Usitatissimum (Linseed) Seed Oil), almond oil (labeling name, INCI: Prunus Amygdalus Dulcis (Sweet Almond) Oil), perilla oil (labeling name), olive oil (labeling name, INCI: Olea Europaea (Olive) Fruit Oil), Torreya California oil (labeling name, INCI: Torreya Californica (California Nutmeg) Oil), citronella grass oil (labeling name, INCI: Cymbopogon Nardus (Citronella) Oil), kaya seed oil (labeling name, INCI: Torreya Nucifera Seed Oil), kyounin oil (labeling name, INCI: Kyounin Yu), sesame oil (labeling name, INCI: Sesamum Indicum (Sesame) Seed Oil), germ oils, such as wheat germ oil (labeling name, INCI: Triticum Vulgare (Wheat) Germ Oil), rice germ oil (labeling name, INCI: Oryza Sativa (Rice) Germ Oil), and corn germ oil (labeling name, INCI: Zea Mays (Corn) Germ Oil), rice bran oil (labeling name, INCI: Oryza Sativa (Rice) Bran Oil), camellia oil (labeling name, INCI: Camellia Kissi Seed Oil), safflower oil (labeling name, INCI: Carthamus Tinctorius (Safflower) Seed Oil), soybean oil (labeling name, INCI: Glycine Soja (Soybean) Oil), gold tea oil (labeling name, INCI: Camellia Sinensis Seed Oil), camellia oil (labeling name, INCI: Camellia Japonica Seed Oil), evening primrose oil (labeling name, INCI: Oenothera Biennis (Evening Primrose) Oil), rapeseed oil (labeling name), persic oil (labeling name), palm oil (labeling name, INCI: Elaeis Guineensis (Palm) Oil), palm kernel oil (labeling name, INCI: Elaeis Guineensis (Palm) Kernel Oil), castor oil (labeling name, INCI: Ricinus Communis (Castor) Seed Oil), sunflower seed oil (labeling name, INCI: Helianthus Annuus (Sunflower) Seed Oil), grape seed oil (labeling name, INCI: Vitis Vinifera (Grape) Seed Oil), jojoba seed oil (labeling name, INCI: Simmondsia Chinensis (Jojoba) Seed Oil), macadamia seed oil (labeling name, INCI: Macadamia Ternifolia Seed Oil), meadowfoam oil (labeling name, INCI: Limnanthes Alba (Meadowfoam) Seed Oil), cotton seed oil (labeling name, INCI: Gossypium Herbaceum (Cotton) Seed Oil), coconut oil (labeling name, INCI: Cocos Nucifera (Coconut) Oil), and peanut oil (labeling name, INCI: Arachis Hypogaea (Peanut) Oil); natural animal oils, such as shark liver oil (labeling name, INCI: Shark Liver Oil), cod liver oil (labeling name, INCI: Cod Liver Oil), fish liver oil (labeling name, INCI: Fish Liver Oil), turtle oil (labeling name, INCI: Turtle Oil), mink oil (labeling name, INCI: Mink Oil), and egg yolk oil (labeling name, INCI: Egg Oil); and semi-synthetic oils and fats, such as hydrogenated coconut oil (labeling name, INCI: Hydrogenated Coconut Oil) and liquid lanolin (labeling name, INCI: Lanolin Oil).

### • Hydrocarbon oils

Examples of the hydrocarbon oils include linear or branched hydrocarbon oils. The hydrocarbon oils may be volatile or nonvolatile. Specific examples include Olefin Oligomers (INCI), isoparaffins, such as (C13,C14) Isoparaffin (INCI), and alkanes, such as Isododecane (INCI), Undecane (INCI), Dodecane (INCI), Isohexadecane (INCI), hydrogenated polyisobutene (labeling name, INCI: Hydrogenated Polyisobutene), Squalane (INCI), Mineral Oil (INCI), Coconut Alkanes (INCI), and (C13-15) Alkane (INCI).

### • Higher alcohols

Examples of the higher alcohols include linear saturated alcohols having 6 or more carbon atoms, such as Lauryl Alcohol (INCI), Hexyldecanol (INCI), Oleyl Alcohol (INCI), Isostearyl Alcohol (INCI), Octyldodecanol (INCI), Decyltetradecanol (INCI), Myristyl Alcohol (INCI), Cetyl Alcohol (INCI), Stearyl Alcohol (INCI), and Behenyl Alcohol (INCI), as well as Batyl Alcohol (INCI). Examples further include sterols, such as Cholesterol (INCI), sitosterol (labeling name, INCI: Beta-Sitosterol), Phytosterols (INCI), and Lanosterol (INCI).

### • Ester oils

Examples of the ester oils include diisobutyl adipate (labeling name, INCI: Diisobutyl Adipate), dihexyldecyl adipate (labeling name), diheptylundecyl adipate (labeling name, INCI: Diheptylundecyl Adipate), alkylglycol monoisostearates, such as isostearyl isostearate (labeling name, INCI: Isostearyl Isostearate), isocetyl isostearate (labeling name, INCI: Isocetyl Isostearate), trimethylolpropane triisostearate (labeling name, INCI: Trimethylolpropane Triisostearate), glycol diethylhexanoate (labeling name, INCI: Glycol Diethylhexanoate), cetyl ethylhexanoate (labeling name, INCI: Cetyl Ethylhexanoate), trimethylolpropane triethylhexanoate (labeling name, INCI: Trimethylolpropane Triethylhexanoate), pentaerythrityl tetraethylhexanoate (labeling name, INCI: Pentaerythrityl Tetraethylhexanoate), octyldodecyl esters, such as octyldodecyl stearoyloxystearate (labeling name, INCI: Octyldodecyl Stearoyl Stearate), oleyl oleate (labeling name, INCI: Oleyl Oleate), octyldodecyl oleate (labeling name, INCI: Octyldodecyl Oleate), decyl oleate (labeling name, INCI: Decyl Oleate), neopentyl glycol dioctanoate (labeling name, INCI: Neopentyl Glycol Diethylhexanoate), neopentyl glycol dicaprate (labeling name, INCI: Neopentyl Glycol Dicaprate), diisostearyl malate (labeling name, INCI: Diisostearyl Malate), triethyl citrate (labeling name, INCI: Triethyl Citrate), diethylhexyl succinate (labeling name, INCI: Diethylhexyl Succinate), amyl acetate (labeling name, INCI: Amyl Acetate), ethyl acetate (labeling name, INCI: Ethyl Acetate), butyl acetate (labeling name, INCI: Butyl Acetate), isocetyl stearate (labeling name, INCI: Isocetyl Stearate), butyl stearate (labeling name, INCI: Butyl Stearate), diisopropyl sebacate (labeling name, INCI: Diisopropyl Sebacate), diethylhexyl sebacate (labeling name, INCI: Diethylhexyl Sebacate), cetyl lactate (labeling name, INCI: Cetyl Lactate), myristyl lactate (labeling name, INCI: Myristyl Lactate), isononyl isononanoate (labeling name, INCI: Isononyl Isononanoate), isotridecyl isononanoate (labeling name, INCI: Isotridecyl Isononanoate), palmitates, such as isopropyl palmitate (labeling name, INCI: Isopropyl Palmitate), ethylhexyl palmitate (labeling name, INCI: Ethylhexyl Isopalmitate), and hexyldecyl palmitate (labeling name, INCI: Isocetyl Palmitate, Hexyldecyl Palmitate), cholesteryl hydroxystearate (labeling name, INCI: Cholesteryl Hydroxystearate), myristates, such as isopropyl myristate (labeling name, INCI: Isopropyl Myristate), octyldodecyl myristate (labeling name, INCI: Octyldodecyl Myristate), and myristyl myristate (labeling name, INCI: Myristyl Myristate), ethylhexyl laurate (labeling name, INCI: Ethylhexyl Laurate), hexyl laurate (labeling name, INCI: Hexyl Laurate), dioctyldodecyl lauroyl glutamate (labeling name, INCI: Dioctyldodecyl Lauroyl Glutamate), isopropyl lauroyl sarcosinate (labeling name, INCI: Isopropyl Lauroyl Sarcosinate), and cocoalkyl (caprylate/caprate) (labeling name, INCI: Coco-Caprylate·Caprate).

Glyceride oils are also included in the ester oils, such as Triethylhexanoin (INCI), glyceryl tri(caprylate/caprate) (labeling name, INCI: Caprylic/Capric Triglyceride), Cocoglyceryl (INCI), triglyceryl (caprylate/caprate/succinate) (labeling name, INCI: Caprylic/Capric/Succinic Triglyceride), and (caprylic/capric) glycerides (labeling name, INCI: Caprylic/Capric Glycerides).

### • Fluorinated oils

Examples of the fluorinated oils include Perfluorodecalin (INCI), Perfluorononyl Dimethicone (INCI), and Perfluoromethylcyclopentane (INCI).

### • UV absorbers

Suitable UV absorbers include oxybenzone-1 (labeling name, INCI: Benzophenone-1), oxybenzone-2 (labeling name, INCI: Benzophenone-2), oxybenzone-3 (labeling name, INCI: Benzophenone-3), oxybenzone-4 (labeling name, INCI: Benzophenone-4), oxybenzone-5 (labeling name, INCI: Benzophenone-5), oxybenzone-6 (labeling name, INCI: Benzophenone-6), oxybenzone-9 (labeling name, INCI: Benzophenone-9), Homosalate (INCI), Octocrylene (INCI), t-butyl methoxydibenzoylmethane (labeling name, INCI: Butyl Methoxydibenzoylmethane), ethyl hexyl salicylate (labeling name, INCI: Ethylhexyl Salicylate), diethylaminohydroxybenzoyl hexyl benzoate (labeling name, INCI: Diethylamino Hydroxybenzoyl Hexyl Benzoate), Polysilicone-15 (INCI), ethylhexyl dimethoxybenzylidene dioxoimidazolidine propionate (labeling name, INCI: Ethylhexyl Dimethoxybenzylidene Dioxoimidazolidine Propionate), terephthalylidene dicamphor sulfonic acid (labeling name, INCI: Terephthalylidene Dicamphor Sulfonic Acid), Ethylhexyl Triazone (INCI), methylbis(trimethylsiloxy)silyl isopentyl trimethoxycinnamate (labeling name, INCI: Isopentyl Trimethoxycinnamate Trisiloxane), Drometrizole Trisiloxane (INCI), dimethyl PABA ethylhexyl (labeling name, INCI: Ethylhexyl Dimethyl PABA), isopropyl para-methoxycinnamate (labeling name, INCI: Isopropyl Methoxycinnamate), ethylhexyl methoxycinnamate (labeling name, INCI: Ethylhexyl Methoxycinnamate), Bis-Ethylhexyloxyphenol Methoxyphenyltriazine (INCI), phenylbenzimidazole sulfonic acid (labeling name, INCI: Phenylbenzimidazole Sulfonic Acid), Methylene Bis-Benzotriazolyl Tetramethylbutylphenol (INCI), glyceryl ethylhexanoate dimethoxycinnamate (labeling name, INCI: Glyceryl Ethylhexanoate Dimethoxycinnamate), Glyceryl PABA (INCI), methyl diisopropylcinnamate (labeling name, INCI: Diisopropyl Methyl Cinnamate), and Cinoxate (INCI). Furthermore, a UVA absorber (such as, for example, diethylamino hydroxybenzoyl hexyl benzoate) and a UVB absorber (such as, for example, ethylhexyl methoxycinnamate) may be used in combination, or absorbers belonging to the same type may be combined appropriately.

### (2) Aqueous components

The aqueous components are not particularly limited as long as they are aqueous components that are usually added to cosmetics. Specific examples include water, lower alcohols preferably having 2 to 5 carbon atoms, such as ethanol (labeling name, INCI: Alcohol) and isopropanol (labeling name, INCI: Isopropyl Alcohol), and sugar alcohols, such as Sorbitol (INCI), Maltose (INCI), and Xylitol (INCI). Examples further include polyhydric alcohols, such as BG (labeling name, INCI: Butylene Glycol), PG (labeling name, INCI: Propylene Glycol), DPG (labeling name, INCI: Dipropylene Glycol), Pentylene Glycol (INCI), 1,10-Decanediol (INCI), Octanediol (INCI), 1,2-Hexanediol (INCI), Erythritol (INCI), Glycerin (INCI), Diglycerin (INCI), and polyethylene glycol; and moisturizers, such as Glucose (INCI), Glyceryl Glucoside (INCI), Betaine (INCI), Na chondroitin sulfate (labeling name, INCI: Sodium Chondroitin Sulfate), PCA-Na (labeling name, INCI: Sodium PCA), Methyl Gluceth-10 (INCI), Methyl Gluceth-20 (INCI), hyaluronic acid, egg yolk lecithin, soybean lecithin, phosphatidylcholine, phosphatidylethanolamine, phosphatidylserine, phosphatidylglycerol, phosphatidylinositol, and sphingophospholipid. Examples further include water-soluble polymer compounds, for example, gum Arabic, guar gum, carrageenan, agar, quince seed gum, locust bean gum, xanthan gum, pullulan, sodium carboxymethyl cellulose, hydroxyethyl cellulose, vinyl polymers, such as carboxyvinyl polymer, and acrylic polymers, such as (ammonium acryloyldimethyltaurate/vinylpyrrolidone) copolymer, (sodium acrylate/sodium acryloyldimethyltaurate) copolymer, (hydroxyethyl acrylate/sodium acryloyldimethyltaurate) copolymer, (acrylamide/sodium acryloyldimethyltaurate) copolymer, and polyacrylamide.

### (3) Surfactants

The surfactants may be nonionic, anionic, cationic, or ampholytic. The surfactants are not particularly limited as long as they are commonly blended in cosmetics. Of the surfactants, one, or two or more surfactants selected from non-crosslinked silicone surfactants and crosslinked silicone surfactants are preferred because a stable cosmetic is obtainable. In any cases of surfactants, the amount is preferably 0.1 to 20% by mass of the overall cosmetic. An amount of at least 0.1% is preferred in that the dispersing and emulsifying functions are fully achieved whereas an amount of up to 20% by mass is preferred because the risk that the cosmetic gives a sticky feeling on use is eliminated. The surfactant preferably has an HLB of 2 to 14.5, though not limitative, for the purpose of maintaining the cosmetic water resistant.

The non-crosslinked silicone surfactants are linear or branched silicones in which some methyl groups on the backbone are substituted with hydrophilic groups, such as polyethylene glycol and polyglycerin. Specifically, preferred such surfactants are linear or branched polyoxyethylene-modified organopolysiloxanes, linear or branched polyoxyethylene polyoxypropylene-modified organopolysiloxanes, linear or branched polyoxyethylene·alkyl-co-modified organopolysiloxanes, linear or branched polyoxyethylene polyoxypropylene·alkyl-co-modified organopolysiloxanes, linear or branched polyglycerin-modified organopolysiloxanes, linear or branched polyglycerin·alkyl-co-modified organopolysiloxanes, and linear or branched pyrrolidone-modified organopolysiloxanes. Examples include PEG-11 Methyl Ether Dimethicone (INCI), PEG/PPG-20/22 Butyl Ether Dimethicone (INCI), PEG-3 Dimethicone (INCI), PEG-10 Dimethicone (INCI), PEG-9 Polydimethylsiloxyethyl Dimethicone (INCI), Lauryl PEG-9 Polydimethylsiloxyethyl Dimethicone (INCI), Cetyl PEG/PPG-10/1 Dimethicone (INCI), Polyglyceryl-3 Disiloxane Dimethicone (INCI), Polyglyceryl-3 Polydimethylsiloxyethyl Dimethicone (INCI), Lauryl Polyglyceryl-3 Polydimethylsiloxyethyl Dimethicone (INCI), and Bis-Butyl Dimethicone Polyglyceryl-3 (INCI).

Commercially available examples include KF-6011, KF-6011P, KF-6012, KF-6015, KF-6017, KF-6043, KF-6028, KF-6038, KF-6048, KF-6100, KF-6104, KF-6106, KF-6105, KF-6115, and KF-6180, manufactured by Shin-Etsu Chemical Co., Ltd.

Examples of the crosslinked silicone surfactants include crosslinked polyether-modified silicones, such as (Dimethicone/(PEG-10/15)) Crosspolymer (INCI), (PEG-15/Lauryl Dimethicone) Crosspolymer (INCI), (PEG-10/Lauryl Dimethicone) Crosspolymer (INCI), and (PEG-15/Lauryl Polydimethylsiloxyethyl Dimethicone) Crosspolymer (INCI); and crosslinked polyglycerin-modified silicones, such as (Dimethicone/Polyglycerin-3) Crosspolymer (INCI), (Lauryl Dimethicone/Polyglycerin-3) Crosspolymer (INCI), and (Polyglycerin-3/Lauryl Polydimethylsiloxyethyl Dimethicone) Crosspolymer (INCI).

On use of the crosslinked silicone surfactant, it is preferred that in a composition consisting of the crosslinked silicone surfactant and an oil that is liquid at room temperature, the crosslinked silicone surfactant swell with the liquid oil as a result of being impregnated with an amount of more than its own weight of the liquid oil. Suitable liquid oils include the silicone oils, the hydrocarbon oils, the ester oils, the natural animal and plant oils, the semi-synthetic oils, and the fluorinated oils that are described hereinabove as the optional components, namely, the oils (1), and are liquid. Illustrative examples include Cyclopentasiloxane (INCI), Dimethicone (INCI), Mineral Oil (INCI), Isododecane (INCI), Isohexadecane (INCI), Triethylhexanoin (INCI), isotridecyl isononanoate (labeling name, INCI: Isotridecyl Isononanoate), and Squalane (INCI). Commercially available examples of the crosslinked silicone surfactants that are swollen with the liquid oil include KSG-210, KSG-240, KSG-270, KSG-310, KSG-320, KSG-330, KSG-340, KSG-320Z, KSG-350Z, KSG-710, KSG-790, KSG-810, KSG-820, KSG-830, KSG-840, KSG-820Z, and KSG-850Z from Shin-Etsu Chemical Co., Ltd.

### (4) Powders other than the inventive powders

Powders other than the powders of the present invention may be added. Examples of such powders include coloring pigments, inorganic powders, metal powders, organic powders, and inorganic/organic composite powders, which are specifically described below.

### • Coloring pigments

The coloring pigments are not particularly limited as long as they are commonly used in cosmetics for the purpose of coloring. Examples include red iron oxide (labeling name, INCI: Iron Oxides), yellow iron oxide (labeling name, INCI: Iron Oxides), white titanium oxide (labeling name, INCI: Titanium Dioxide), black iron oxide (labeling name, INCI: Iron Oxides), Ultramarine (labeling name, INCI: Ultramarines), Prussian blue (labeling name, INCI: Ferric Ferrocyanide, Ferric Ammonium Ferrocyanide), manganese violet (labeling name, INCI: Manganese Violet), cobalt titanate (labeling name, INCI: Cobalt Titanium Oxide), chromium hydroxide (labeling name, INCI: Chromium Hydroxide Green), chromium oxide (labeling name, INCI: Chromium Oxide Greens), Al/cobalt oxide (labeling name, INCI: Cobalt Aluminum Oxide), fired titanium/titanium oxide (labeling name, INCI: Titanium/Titanium Dioxide), Li/cobalt titanate (labeling name, INCI: Lithium Cobalt Titanate), sintered iron oxide/titanium oxide (labeling name), composites doped with a different metal, such as iron oxide-doped titanium oxide (labeling name, INCI: Iron Oxides, Titanium Dioxide), titanium nitride (labeling name, INCI: Titanium Nitride), ferrous hydroxide (labeling name, INCI: Iron Hydroxide), inorganic brown pigments, such as γ-iron oxide, inorganic yellow pigments, such as loess, and colored pigments, such as lake form of tar dyes and lake form of natural dyes. Any of the foregoing may be used.

The pigment may take any shape including spherical, generally spherical, bar, spindle, petal, strip, and irregular shapes. Its geometry is not particularly limited as long as a color can be imparted to the cosmetic.

### • Inorganic powders

Examples of the inorganic powders include microparticles of zirconium oxide (labeling name, INCI: Zirconium Dioxide), zinc oxide (labeling name, INCI: Zinc Oxide), cerium oxide (labeling name, INCI: Cerium Oxide), magnesium oxide (labeling name, INCI: Magnesium Oxide), barium sulfate (labeling name, INCI: Barium Sulfate), calcium sulfate (labeling name, INCI: Calcium Sulfate), magnesium sulfate (labeling name, INCI: Magnesium Sulfate), calcium carbonate (labeling name, INCI: Calcium Carbonate), magnesium carbonate (labeling name, INCI: Magnesium Carbonate), Talc (INCI), Mica (INCI), Kaolin (INCI), synthetic fluorphlogopite (labeling name, INCI: Synthetic Fluorphlogopite), synthetic ferrous golden mica (labeling name), black mica (labeling name, INCI: Biotite), potassium silicate (labeling name, INCI: Potassium Silicate), Silica (INCI), aluminum silicate (labeling name, INCI: Aluminum Silicate), magnesium silicate (labeling name, INCI: Magnesium Silicate), aluminum/magnesium silicate (labeling name, INCI: Magnesium Aluminum Silicate), calcium silicate (labeling name, INCI: Calcium Silicate), aluminum/calcium/sodium silicate (labeling name, INCI: Aluminum Calcium Sodium Silicate), lithium/magnesium/sodium silicate (labeling name, INCI: Lithium Magnesium Sodium Silicate), sodium/magnesium silicate (labeling name, INCI: Sodium Magnesium Silicate), calcium/aluminum borosilicate (labeling name, INCI: Calcium Aluminum Borosilicate), calcium/sodium borosilicate (labeling name, INCI: Calcium Sodium Borosilicate), Hydroxyapatite (INCI), Bentonite (INCI), Montmorillonite (INCI), Hectorite (INCI), Zeolite (INCI), Alumina (INCI), aluminum hydroxide (labeling name, INCI: Aluminum Hydroxide), boron nitride (labeling name, INCI: Boron Nitride), and glass (labeling name, INCI: Glass). Also, examples of the inorganic coloring pearly pigments include pearly agents, such as Mica (INCI) coated with titanium oxide (labeling name, INCI: Titanium dioxide), and synthetic fluorphlogopite (labeling name, INCI: Synthetic Fluorphlogopite) coated with titanium oxide (labeling name, INCI: Titanium Dioxide); and pearly pigments, such as bismuth oxychloride (labeling name, INCI: Bismuth Oxychloride), bismuth oxychloride (labeling name, INCI: Bismuth Oxychloride) coated with titanium oxide (labeling name, INCI: Titanium Dioxide), Talc (INCI) coated with titanium oxide (labeling name, INCI: Titanium Dioxide), fish scale flakes (labeling name), and coloring mica coated with titanium oxide (labeling name, INCI: Titanium Dioxide). These may either be untreated or have undergone well-known surface treatment commonly used for cosmetics.

### • Metal powders

Examples of the metal powders include metal microparticles, such as aluminum (labeling name, INCI: Aluminum, Aluminum Powder), copper (labeling name, INCI: Copper Powder), silver (labeling name, INCI: Silver Powder), and gold (labeling name, INCI: Gold).

### • Organic powders

Examples of the organic powders include powders of silicones, polyamides, polyacrylic acid-acrylate, polyesters, Polyethylene (INCI), Polypropylene (INCI), Polystyrene (INCI), styrene-acrylic acid copolymers, divinylbenzene-styrene copolymers, polyurethane, vinyl resins, urea resins, melamine resins, benzoguanamine, polymethyl benzoguanamine, tetrafluoroethylene, polymethyl methacrylate, Cellulose (INCI), Silk (INCI), nylon (labeling name), phenolic resins, epoxy resins, and polycarbonate.

In particular, examples of the silicones include silicone resin particles; Polymethylsilsesquioxane (INCI), silicone rubber powder, silicone resin-coated silicone rubber powder; (Vinyl Dimethicone/Methicone Silsesquioxane) Crosspolymer (INCI), (Diphenyl Dimethicone/Vinyl Diphenyl Dimethicone/Silsesquioxane) Crosspolymer (INCI), Polysilicone-1 Crosspolymer (INCI), and Polysilicone-22 (INCI).

Commercially available examples of the silicone powders include KMP-590, KMP-591, KMP-592, KMP-597, KMP-598, KSP-100, KSP-101, KSP-102, KSP-105, KSP-300, KSP-411, KSP-441, KM-9729, and KM-440, manufactured by Shin-Etsu Chemical Co., Ltd.

Examples further include metal soaps. Specific examples include powders of zinc stearate (labeling name, INCI: Zinc Stearate), aluminum stearate (labeling name, INCI: Aluminum Stearate), calcium stearate (labeling name, INCI: Calcium Stearate), magnesium stearate (labeling name, INCI: Magnesium Stearate), zinc myristate (labeling name, INCI: Zinc Myristate), magnesium myristate (labeling name, INCI: Magnesium Myristate), zinc/sodium cetylphosphate (labeling name, INCI: Sodium Zinc Cetyl Phosphate), and potassium cetylphosphate (labeling name, INCI: Potassium Cetyl Phosphate). Examples further include organic colorants. Specific examples include tar dyes, such as Red #3, Red #104 (1) (labeling name, INCI: Red 28, Red 28 Lake), Red #106, Red #201 (labeling name, INCI: Red 6), Red #202 (labeling name, INCI: Red 7), Red #204, Red #205, Red #220 (labeling name, INCI: Red 34), Red #226 (labeling name, INCI: Red 30), Red #227 (labeling name, INCI: Red 33, Red 33 Lake), Red #228 (labeling name, INCI: Red 36), Red #230 (1) (labeling name, INCI: Red 22, Red 22 Lake), Red #230 (2) (labeling name), Red #401 (labeling name), Red #505 (labeling name), Yellow #4 (labeling name, INCI: Yellow 5), Yellow #5 (labeling name, INCI: Yellow 6, Yellow 6 Lake), Yellow #202 (1) (labeling name, INCI: Yellow 8), Yellow #203 (labeling name, INCI: Yellow 10, Yellow 10 Lake), Yellow #204 (labeling name, INCI: Yellow 11), Yellow #401, Blue #1 (labeling name, INCI: Blue 1, Blue 1 Lake), Blue #2, Blue #201, Blue #205 (labeling name, INCI: Blue 4), Blue #404 (labeling name), Green #3 (labeling name, INCI: Green 3, Green 3 Lake), Green #201 (labeling name, INCI: Green 5), Green #202 (labeling name, INCI: Green 6), Green #204 (labeling name, INCI: Green 8), Green #205 (labeling name), Orange #201 (labeling name, INCI: Orange 5), Orange #203 (labeling name, INCI: Pigment Orange 5), Orange #204 (labeling name), Orange #205 (labeling name, INCI: Orange 4, Orange 4 Lake), Orange #206 (labeling name, INCI: Orange 10), and Orange #207 (labeling name, INCI: Orange 11); and natural dyes, such as Cochineal (INCI), laccaic acid (labeling name, INCI: Laccaic Acid), safflower red (labeling name, INCI: Carthamus Tinctorius (Safflower) Flower Extract), red root extract (labeling name, INCI: Lithospermum Officinale Root Extract), gardenia yellow (labeling name), and gardenia blue (labeling name, INCI: Hydrolyzed Gardenia Florida Extract).

### • Inorganic/organic composite powders

Exemplary of the inorganic/organic composite powders is a composite powder obtained by coating the surface of an inorganic powder with an organic powder by any well-known method.

The powders described above may be used as having been treated on the surface of particles. The surface treating agent is preferably one capable of imparting hydrophobicity from the aspect of water resistance of the cosmetic. The hydrophobizing agent is not particularly limited. Exemplary treating agents include silicone treating agents, waxes, paraffins, organic fluorine compounds, such as perfluoroalkyl phosphate salts, surfactants, amino acids, such as N-acylglutamic acid, and metal soaps, such as aluminum stearate and magnesium myristate. The silicone treating agents are more preferable, with examples including silanes or silylating agents, such as Triethoxycaprylylsilane (INCI); Dimethicone (INCI), Methicone (INCI), Hydrogen Dimethicone (INCI), Triethoxysilylethyl Polydimethylsiloxyethyl Dimethicone (INCI), Triethoxysilylethyl Polydimethylsiloxyethylhexyl Dimethicone (INCI), and (acrylates/tridecyl acrylate/triethoxysilylpropyl methacrylate/dimethicone methacrylate) copolymer (labeling name, INCI: Acrylates/Tridecyl Acrylate/Triethoxysilylpropyl Methacrylate/Dimethicone Methacrylate Copolymer). Specific examples of the silicone treating agents include AES-3083, KF-99P, KF-9901, KF-9908, KF-9909, KP-574, and KP-541, manufactured by Shin-Etsu Chemical Co., Ltd. The surface hydrophobizing agents may be used singly, or two or more may be used in combination. Specific examples of the surface-treated coloring pigments include KTP-09 series, especially KTP-09W, 09R, 09Y, and 09B, from Shin-Etsu Chemical Co., Ltd.

### (5) Compositions consisting of a crosslinked organopolysiloxane and an oil that is liquid at room temperature

In the composition consisting of a crosslinked organopolysiloxane and an oil that is liquid at room temperature, it is preferred that the crosslinked organopolysiloxane swell with the liquid oil as a result of being impregnated with an amount of more than its own weight of the liquid oil. Suitable liquid oils include the silicone oils, the hydrocarbon oils, the ester oils, the natural animal and plant oils, the semi-synthetic oils, and the fluorinated oils that are described hereinabove as the optional components, namely, the oils (1), and are liquid. Illustrative examples include Cyclopentasiloxane (INCI), Dimethicone (INCI), Mineral Oil (INCI), Isododecane (INCI), Isohexadecane (INCI), Triethylhexanoin (INCI), isotridecyl isononanoate (labeling name, INCI: Isotridecyl Isononanoate), and Squalane (INCI).

Unlike the crosslinked silicone surfactants as the components (3) mentioned above, the components (5) are compounds having neither polyether nor polyglycerin structure in the molecular structure. Specific examples include Dimethicone/Vinyl Dimethicone Crosspolymer (INCI), Dimethicone/Phenyl Vinyl Dimethicone Crosspolymer (INCI), Vinyl Dimethicone/Lauryl Dimethicone Crosspolymer (INCI), and Lauryl Polydimethylsiloxyethyl Dimethicone/Bis-Vinyl Dimethicone Crosspolymer (INCI). Commercially available examples of the compositions consisting of a crosslinked organopolysiloxane and an oil that is liquid at room temperature include KSG-15, KSG-1510, KSG-16, KSG-1610, KSG-19, KSG-016F, KSG-18A, KSG-41A, KSG-42A, KSG-43, KSG-44, KSG-45, KSG-042Z, KSG-045Z, and KSG-048Z, from Shin-Etsu Chemical Co., Ltd.

### (6) Film-forming agents

The film-forming agent is blended mainly for the purpose of further maintaining the effect-sustainability of the cosmetic. A silicone composition is preferred from the aspect of imparting water repellency, though the agent is not limited thereto. Specifically, use may be made of, for example, trimethylsiloxysilicate, acrylic-silicone film formers, silicone-modified norbornene, silicone-modified pullulan, and silicone-modified polyvinyl alcohol. Examples of the film-forming agents in the form of silicone composition include trimethylsiloxysilicate (labeling name, INCI: Trimethylsiloxysilicate), Acrylates/Dimethicone Copolymer (INCI), Norbornene/Tris-(Trimethylsiloxy)Silylnorbornene Copolymer (INCI), and pullulan tri(trimethylsiloxy)silylpropyl carbamide (labeling name, INCI: Trimethylsiloxysilylcarbamoyl Pullulan). The film-forming agent may be previously dissolved in an oil that is liquid at room temperature before it is blended in the cosmetic. Suitable liquid oils used herein include the silicone oils, the hydrocarbon oils, the ester oils, the natural animal and plant oils, the semi-synthetic oils, and the fluorinated oils that are described hereinabove as the optional components, namely, the oils (1), and are liquid. Specific examples of the commercially available silicone film-forming agents include K-7312J, KP-545, KP-549, KP-543, NBN-30-ID, TSPL-30-ID, and TSPL-30-D5, from Shin-Etsu Chemical Co., Ltd.

### (7) UV absorbing/scattering agents

Examples of the UV absorbing/scattering agents include particles that absorb and scatter ultraviolet rays, such as titanium oxide microparticles, iron-containing titanium oxide microparticles, zinc oxide microparticles, cerium oxide microparticles, and composites thereof. These UV absorbing/scattering particles may be used as a dispersion in an oil. The oil may be any of the silicone oils, the hydrocarbon oils, the ester oils, the natural animal and plant oils, the semi-synthetic oils, and the fluorinated oils that are described hereinabove as the optional components, namely, the oils (1), and are liquid. Specific examples of the oil dispersions of the UV absorbing/scattering particles include SPD series (product name), in particular, SPD-T5, T5L, Z5, Z5L, T6, Z6, T7, and Z7L, manufactured by Shin-Etsu Chemical Co., Ltd.

### (8) Other additives

Other additives include oil-soluble gelling agents, preservatives, antibacterial agents, antiperspirants, fragrances, salts, antioxidants, acidity regulators, chelating agents, refreshing agents, anti-inflammatory agents, skin modifying ingredients (e.g., brightening or whitening agents, cell activators, anti-skin-roughening agents, blood flow enhancing agents, skin astringents, antiseborrheic agents), vitamins, amino acids, nucleic acids, hormones, and clathrate compounds.

### • Oil-soluble gelling agents

Exemplary oil-soluble gelling agents include metal soaps, such as aluminum stearate, magnesium stearate, and zinc myristate; amino acid derivatives, such as lauroyl glutamic acid (labeling name, INCI: Lauroyl Glutamic Acid ) and α,γ-di-n-butylamine; dextrin fatty acid esters, such as dextrin palmitate (labeling name, INCI: Dextrin Palmitate), dextrin isostearate (labeling name, INCI: Dextrin Isostearate), dextrin myristate (labeling name, INCI: Dextrin Myristate), inulin stearate (labeling name, INCI: Stearoyl Inulin), and dextrin palmitate/ethylhexanoate (labeling name, INCI: Dextrin Palmitate/Ethylhexanoate); sucrose fatty acid esters, such as sucrose palmitate and sucrose stearate; fructooligosaccharide fatty acid esters, such as fructooligosaccharide stearate and fructooligosaccharide 2-ethylhexanoate; benzylidene derivatives of sorbitol, such as monobenzylidene sorbitol and dibenzylidene sorbitol; organo-modified clay minerals, such as Disteardimonium Hectorite (INCI), Stearalkonium Hectorite (INCI), and hectorite; and Stearalkonium Bentonite (INCI).

### • Preservatives/antibacterial agents

Suitable preservatives and antibacterial agents include alkyl p-hydroxybenzoates, benzoic acid, sodium benzoate, sorbic acid, potassium sorbate, phenoxyethanol, imidazolidinyl urea, salicylic acid, isopropyl methyl phenol, phenol, p-chloro-m-cresol, hexachlorophene, benzalkonium chloride, chlorohexidine chloride, trichlorocarbanilide, iodopropynyl butylcarbamate, polylysine, photosensitizers, silver, and plant extracts.

### • Antiperspirants

Examples of the antiperspirants include aluminum halohydrates, such as aluminum chlorohydrate, aluminum halides, such as aluminum chloride, aluminum allantoinate, tannic acid, persimmon tannin, aluminum potassium sulfate, zinc oxide, zinc p-phenolsulfonate, aluminum potassium sulfate, aluminum zirconium tetrachlorohydrate, and aluminum zirconium trichlorohydrex glycine. Preferred components that exert a higher effect include aluminum halohydrates, aluminum halides, and complexes or mixtures thereof with zirconium oxyhalides and zirconium hydroxyhalides (e.g., aluminum zirconium tetrachlorohydrate and aluminum zirconium trichlorohydrex glycine).

### • Fragrances

Examples of the fragrances include natural fragrances and synthetic fragrances. Suitable natural fragrances include plant fragrances separated from flowers, leaves, stems, pericarps, and the like; and animal fragrances, such as musk and civet. Suitable synthetic fragrances include hydrocarbons, such as monoterpene; alcohols, such as aliphatic alcohols and aromatic alcohols; aldehydes, such as terpene aldehydes and aromatic aldehydes; ketones, such as alicyclic ketones; esters, such as terpene esters; lactones; phenols; oxides; nitrogen-containing compounds; and acetals.

### • Salts

Examples of the salts include inorganic salts, organic acid salts, amine salts, and amino acid salts. Exemplary inorganic salts include sodium salts, potassium salts, magnesium salts, calcium salts, aluminum salts, zirconium salts, and zinc salts of inorganic acids, such as hydrochloric acid, sulfuric acid, carbonic acid, and nitric acid. Exemplary organic acid salts include salts of organic acids, such as acetic acid, dehydroacetic acid, citric acid, malic acid, succinic acid, ascorbic acid, and stearic acid. Exemplary amine salts and amino acid salts include salts of amines, such as triethanol amine, and salts of amino acids, such as glutamic acid. Also useful are salts of hyaluronic acid, chondroitin sulfate, aluminum zirconium glycine complex, and acid-alkali neutral salts used in cosmetic formulations.

### • Antioxidants

Examples of the antioxidants include, but are not particularly limited to, carotenoid, ascorbic acid and salts thereof, ascorbyl stearate, tocophenol, tocophenol acetate, tocopherol, p-t-butylphenol, butyl hydroxyanisole, dibutyl hydroxytoluene, phytic acid, ferulic acid, thiotaurine, hypotaurine, sulfites, erythorbic acid and salts thereof, chlorogenic acid, epicatechin, epigallocatechin, epigallocatechin gallate, apigenin, kaempferol, myricetin, and quercetin. The antioxidants may be used singly, or two or more may be used in combination.

### • Acidity regulators

Suitable acidity regulators include lactic acid, citric acid, glycolic acid, succinic acid, tartaric acid, dl-malic acid, potassium carbonate, sodium hydrogencarbonate, and ammonium hydrogencarbonate.

### • Chelating agents

Suitable chelating agents include alanine, sodium EDTA, sodium polyphosphate, sodium metaphosphate, and phosphoric acid.

### • Refreshing agents

Suitable refreshing agents include L-menthol, camphor, and menthyl lactate.

### • Anti-inflammatory agents

Suitable anti-inflammatory agents include allantoin, glycyrrhizinic acid and salts thereof, glycyrrhetinic acid, stearyl glycyrrhetinate, tranexamic acid, and azulene.

### • Skin modifying ingredients

Suitable skin modifying ingredients include brightening or whitening agents, such as arbutin, glutathione, and Saxifraga Sarmentosa Extract; wrinkle improving agents, such as retinol and niacinamide; cell activators, such as royal jelly, photosensitizers, cholesterol derivatives, and bovine blood extracts; anti-skin-roughening agents; blood flow enhancing agents, such as vanillylamide nonanoate, benzyl nicotinate, β-butoxyethyl nicotinate, capsaicin, zingerone, cantharides tincture, ichthammol, caffeine, tannic acid, α-borneol, tocopherol nicotinate, inositol hexanicotinate, cyclandelate, cinnarizine, tolazoline, acetylcholine, verapamil, cepharanthine, and γ-oryzanol; skin astringents, such as zinc oxide and tannic acid; and antiseborrheic agents, such as sulfur and thianthol.

### • Vitamins

Suitable vitamins include vitamin A family, such as vitamin A oil, retinol, retinol acetate, and retinol palmitate; vitamin B family, specifically, vitamin B2 family, such as riboflavin, riboflavin butyrate, and flavin adenine nucleotide, vitamin B6 family, such as pyridoxine hydrochloride, pyridoxine dioctanoate, and pyridoxine tripalmitate, vitamin B12 and derivatives thereof, and vitamin B15 and derivatives thereof; vitamin C family, such as L-ascorbic acid, L-ascorbic acid dipalmitic acid ester, L-ascorbic acid-2-sodium sulfate, and dipotassium L-ascorbic acid phosphoric acid diester; vitamin D family, such as ergocalciferol and cholecalciferol; vitamin E family, such as α-tocopherol, β-tocopherol, γ-tocopherol, dl-α-tocopherol acetate, dl-α-tocopherol nicotinate, and dl-α-tocopherol succinate; nicotinic acids, such as nicotinic acid, benzyl nicotinate, and nicotinic amide; vitamin H; vitamin P; pantothenic acids, such as calcium pantothenate, D-pantothenyl alcohol, pantothenyl ethyl ether, and acetylpantothenyl ethyl ether; and biotin.

### • Amino acids

Suitable amino acids include glycine, valine, leucine, isoleucine, serine, threonine, phenylalanine, arginine, lysine, aspartic acid, glutamic acid, cystine, cysteine, methionine, and tryptophan.

### • Nucleic acids

Typical of the nucleic acids is deoxyribonucleic acid.

### • Hormones

Suitable hormones include estradiol and ethenyl estradiol.

### • Clathrate compounds

Typical of the clathrate compounds is cyclodextrin.

### EXAMPLES

The present invention will be described in detail below by presenting Examples and Comparative Examples. However, the present invention is not limited to the following Examples. The amount of product name is the amount of the product blended.

### <I. Examples 1 to 5: Silica-coated plate-like powders coated with a layer of mesoporous silica>

### [Example 1]

A 200 mL reaction vessel was charged with 11.3 g of mica (Y-2300, manufactured by YAMAGUCHI MICA CO., LTD.), 211.8 g of water, 0.638 g of dodecyltrimethylammonium chloride (a concentration approximately 1 time the critical micelle concentration), and 0.06 g of 29% by mass aqueous ammonia. The mixture was cooled to 5 to 10°C, and 1.238 g of tetramethoxysilane (concentration: 33 mmol/L) was added dropwise. The reaction was performed at 5 to 10°C for 4 hours. After the reaction, the temperature of the solution was raised to 25°C. Subsequently, 12.5 g of acetic acid was added to lower the pH to about 3, and the mixture was reacted at 25°C for 16 hours. After the reaction, the precipitate was filtered, washed with water, and dried under reduced pressure for 3 hours under conditions of 100°C and -100 kPa. Mesoporous silica layer-coated mica (M1-1, mesoporous silica layer: 11% by mass) was thus obtained.

### [Example 2]

A 200 mL reaction vessel was charged with 11.3 g of mica (Y-2300, manufactured by YAMAGUCHI MICA CO., LTD.), 208.4 g of water, 0.638 g of dodecyltrimethylammonium chloride (a concentration approximately 1 time the critical micelle concentration), and 0.06 g of 29% by mass aqueous ammonia. The mixture was cooled to 5 to 10°C, and 4.635 g of tetramethoxysilane (concentration: 135 mmol/L) was added dropwise. The reaction was performed at 5 to 10°C for 4 hours. After the reaction, the temperature of the solution was raised to 25°C. Subsequently, 12.5 g of acetic acid was added to lower the pH to about 3, and the mixture was reacted at 25°C for 16 hours. After the reaction, the precipitate was filtered, washed with water, and dried under reduced pressure for 3 hours under conditions of 100°C and -100 kPa. Mesoporous silica layer-coated mica (M1-2, mesoporous silica layer: 41% by mass) was thus obtained.

### [Example 3]

A 200 mL reaction vessel was charged with 11.3 g of mica (Y-2300, manufactured by YAMAGUCHI MICA CO., LTD.), 206.4 g of water, 0.638 g of dodecyltrimethylammonium chloride (a concentration approximately 1 time the critical micelle concentration), and 0.06 g of 29% by mass aqueous ammonia. The mixture was cooled to 5 to 10°C, and 6.638 g of tetramethoxysilane (concentration: 194 mmol/L) was added dropwise. The reaction was performed at 5 to 10°C for 4 hours. After the reaction, the temperature of the solution was raised to 25°C. Subsequently, 12.5 g of acetic acid was added to lower the pH to about 3, and the mixture was reacted at 25°C for 16 hours. After the reaction, the precipitate was filtered, washed with water, and dried under reduced pressure for 3 hours under conditions of 100°C and -100 kPa. Mesoporous silica layer-coated mica (M1-3, mesoporous silica layer: 59% by mass) was thus obtained.

### [Example 4]

A 200 mL reaction vessel was charged with 11.3 g of mica (Y-2300, manufactured by YAMAGUCHI MICA CO., LTD.), 211.8 g of water, 0.638 g of dodecyltrimethylammonium chloride (a concentration approximately 1 time the critical micelle concentration), and 0.06 g of 29% by mass aqueous ammonia. The mixture was cooled to 5 to 10°C, and 1.238 g of tetramethoxysilane (concentration: 33 mmol/L) was added dropwise. The reaction was performed at 5 to 10°C for 4 hours. After the reaction, the temperature of the solution was raised to 25°C. Subsequently, 0.125 g of acetic acid was added to lower the pH to about 3, and the mixture was reacted at 25°C for 1 hour. After the reaction, the precipitate was filtered, washed with water, and dried under reduced pressure for 3 hours under conditions of 100°C and -100 kPa. Mesoporous silica layer-coated mica (M1-4, mesoporous silica layer: 11% by mass) was thus obtained.

### [Example 5]

A 200 mL reaction vessel was charged with 11.3 g of mica (Y-2300, manufactured by YAMAGUCHI MICA CO., LTD.), 206.4 g of water, 0.638 g of dodecyltrimethylammonium chloride (a concentration approximately 1 time the critical micelle concentration), and 0.06 g of 29% by mass aqueous ammonia. The mixture was cooled to 5 to 10°C, and 6.638 g of tetramethoxysilane (concentration: 194 mmol/L) was added dropwise. The reaction was performed at 5 to 10°C for 4 hours. After the reaction, the temperature of the solution was raised to 25°C. Subsequently, 62.5 g of acetic acid was added to lower the pH to about 3, and the mixture was reacted at 25°C for 72 hours. After the reaction, the precipitate was filtered, washed with water, and dried under reduced pressure for 3 hours under conditions of 100°C and -100 kPa. Mesoporous silica layer-coated mica (M1-5, mesoporous silica layer: 59% by mass) was thus obtained.

### <Synthesis Examples 1-5: Spherical silicas (sol-gel spherical silicas)>

### [Synthesis Example 1]

A 1 L reaction vessel was charged with 108.5 g of ethanol and 9.9 g of 24.5% by mass aqueous ammonia. The mixture was heated to 60°C. Subsequently, 273.5 g of tetraethoxysilane and 68.7 g of 5.6% by mass aqueous ammonia were added dropwise over 45 minutes. The reaction was performed at 60°C for 1 hour. After the reaction, 500 mL of water was added and the temperature was raised to 100°C to replace the solvent with water. The solution was then filtered to give a dispersion of sol-gel silica particles (S-1) (solid concentration: 16.0% by mass).

### [Synthesis Example 2]

A 1 L reaction vessel was charged with 108.5 g of ethanol and 9.9 g of 24.5% by mass aqueous ammonia. The mixture was heated to 60°C. Subsequently, 273.5 g of tetraethoxysilane and 68.7 g of 5.6% by mass aqueous ammonia were added dropwise over 1 hour and 10 minutes. The reaction was performed at 60°C for 1 hour. After the reaction, 500 mL of water was added and the temperature was raised to 100°C to replace the solvent with water. The solution was then filtered to give a dispersion of sol-gel silica particles (S-2) (solid concentration: 15.0% by mass). Furthermore, 50 g of the dispersion of sol-gel silica particles S-2 was added to a 100 mL vessel and was freeze-dried to remove water. Thus, 7.5 g of the powder (S-2-2) of S-2 was obtained.

### [Synthesis Example 3]

A 1 L reaction vessel was charged with 108.5 g of ethanol and 9.9 g of 24.5% by mass aqueous ammonia. The mixture was heated to 55°C. Subsequently, 273.5 g of tetraethoxysilane and 68.7 g of 5.6% by mass aqueous ammonia were added dropwise over 2 hours. The reaction was performed at 55°C for 1 hour. After the reaction, 500 mL of water was added and the temperature was raised to 100°C to replace the solvent with water. The solution was then filtered to give a dispersion of sol-gel silica particles (S-3) (solid concentration: 18.0% by mass).

### [Synthesis Example 4]

A 3 L reaction vessel was charged with 311.9 g of methanol, 21.2 g of 28% by mass aqueous ammonia, and 24.4 g of water. The mixture was heated to 42°C. Subsequently, 561.9 g of tetramethoxysilane and 209.1 g of 4.4% by mass aqueous ammonia were added dropwise. The reaction was performed at 40°C for 1 hour. After the reaction, 600 mL of water was added and the temperature was raised to 100°C to replace the solvent with water. The solution was then filtered to give a dispersion of sol-gel silica particles (S-4) (solid concentration: 12.0% by mass).

### [Synthesis Example 5]

A 1 L reaction vessel was charged with 108.5 g of ethanol and 9.9 g of 24.5% by mass aqueous ammonia. The mixture was heated to 40°C. Subsequently, 273.5 g of tetraethoxysilane and 68.7 g of 5.6% by mass aqueous ammonia were added dropwise over 30 minutes. The reaction was performed at 40°C for 1 hour. After the reaction, 500 mL of water was added and the temperature was raised to 100°C to replace the solvent with water. The solution was then filtered to give a dispersion of sol-gel silica particles (S-5) (solid concentration: 15.0% by mass).

### <Measurement of the average particle diameter (volume median diameter (D50)) and the circularity of the spherical silicas>

The sol-gel silica particles S-1 to S-5 obtained in Synthesis Examples 1 to 5 were analyzed on a dynamic light scattering particle size distribution analyzer (Nanotrac Wave II-Ex150, manufactured by MicrotracBEL Corp.) to determine the average particle diameter. To determine the circularity, the particles were photographed with a scanning electron microscope (SEM), and the major axis and the minor axis of randomly selected 30 particles were measured to calculate the minor axis/major axis ratio of each particle. The minor axis/major axis ratios were averaged to give the circularity.

The measurement results of median diameter and circularity are described in Table 1 below.

**[Table 1]**

| Sol-gel spherical silica | | Average particle e diameter | Circularity |
|---|---|---|---|
| Synthesis Example 1 | S-1 | 150 | 0.92 |
| Synthesis Example 2 | S-2 | 300 | 0.93 |
| Synthesis Example 3 | S-3 | 450 | 0.94 |
| Synthesis Example 4 | S-4 | 80 | 0.87 |
| Synthesis Example 5 | S-5 | 550 | 0.94 |

### <Examples 6 to 14: Synthesis of immobilized spherical silica-bearing silica-coated composite powders>

### [Example 6]

A 200 mL reaction vessel was charged with 176.3 g of water, 11.3 g of the mesoporous silica layer-coated mica (M1-2) prepared in Example 2, 0.638 g of dodecyltrimethylammonium chloride (a concentration approximately 1 time the critical micelle concentration), 13.1 g of the dispersion of the sol-gel silica (S-2) (solid concentration: 15.0% by mass) prepared in Synthesis Example 2, and 0.06 g of 29% by mass aqueous ammonia. The mixture was cooled to 5 to 10°C. Subsequently, 3.09 g of tetramethoxysilane (concentration: 90 mmol/L) was added dropwise. The reaction was performed at 5 to 10°C for 4 hours. After the reaction, the temperature of the solution was raised to 25°C. Subsequently, 11.2 g of acetic acid was added to lower the pH to about 3, and the mixture was reacted at 25°C for 16 hours. After the reaction, the precipitate was filtered, washed with water, and dried under reduced pressure for 3 hours under conditions of 100°C and -100 kPa. Thus, 12 g of a composite powder of M1-2 and S-2 was obtained (the weight of the sol-gel silica particles was 17% by mass relative to the mesoporous silica layer-coated mica).

### [Example 7]

A 200 mL reaction vessel was charged with 176.0 g of water, 11.6 g of the mesoporous silica layer-coated mica (M1-2) prepared in Example 2, 0.638 g of dodecyltrimethylammonium chloride (a concentration approximately 1 time the critical micelle concentration), 13.1 g of the dispersion of the sol-gel silica (S-1) (solid concentration: 16.0% by mass) prepared in Synthesis Example 1, and 0.06 g of 29% by mass aqueous ammonia. The mixture was cooled to 5 to 10°C. Subsequently, 3.09 g of tetramethoxysilane (concentration: 90 mmol/L) was added dropwise. The reaction was performed at 5 to 10°C for 4 hours. After the reaction, the temperature of the solution was raised to 25°C. Subsequently, 11.2 g of acetic acid was added to lower the pH to about 3, and the mixture was reacted at 25°C for 16 hours. After the reaction, the precipitate was filtered, washed with water, and dried under reduced pressure for 3 hours under conditions of 100°C and -100 kPa. Thus, 13 g of a composite powder of M1-2 and S-1 was obtained (the weight of the sol-gel silica particles was 17% by mass relative to the mesoporous silica layer-coated mica).

### [Example 8]

A 200 mL reaction vessel was charged with 178.5 g of water, 11.6 g of the mesoporous silica layer-coated mica (M1-2) prepared in Example 2, 0.638 g of dodecyltrimethylammonium chloride (a concentration approximately 1 time the critical micelle concentration), 10.9 g of the dispersion of the sol-gel silica (S-3) (solid concentration: 18.0% by mass) prepared in Synthesis Example 3, and 0.06 g of 29% by mass aqueous ammonia. The mixture was cooled to 5 to 10°C. Subsequently, 3.09 g of tetramethoxysilane (concentration: 90 mmol/L) was added dropwise. The reaction was performed at 5 to 10°C for 4 hours. After the reaction, the temperature of the solution was raised to 25°C. Subsequently, 11.2 g of acetic acid was added to lower the pH to about 3, and the mixture was reacted at 25°C for 16 hours. After the reaction, the precipitate was filtered, washed with water, and dried under reduced pressure for 3 hours under conditions of 100°C and -100 kPa. Thus, 12 g of a composite powder of M1-2 and S-3 was obtained (the weight of the sol-gel silica particles was 17% by mass relative to the mesoporous silica layer-coated mica).

### [Example 9]

A 200 mL reaction vessel was charged with 173.0 g of water, 11.6 g of the mesoporous silica layer-coated mica (M1-2) prepared in Example 2, 0.638 g of dodecyltrimethylammonium chloride (a concentration approximately 1 time the critical micelle concentration), 16.4 g of the dispersion of the sol-gel silica (S-4) (solid concentration: 12.0% by mass) prepared in Synthesis Example 4, and 0.06 g of 29% by mass aqueous ammonia. The mixture was cooled to 5 to 10°C. Subsequently, 3.09 g of tetramethoxysilane (concentration: 90 mmol/L) was added dropwise. The reaction was performed at 5 to 10°C for 4 hours. After the reaction, the temperature of the solution was raised to 25°C. Subsequently, 11.2 g of acetic acid was added to lower the pH to about 3, and the mixture was reacted at 25°C for 16 hours. After the reaction, the precipitate was filtered, washed with water, and dried under reduced pressure for 3 hours under conditions of 100°C and -100 kPa. Thus, 13 g of a composite powder of M1-2 and S-4 was obtained (the weight of the sol-gel silica particles was 17% by mass relative to the mesoporous silica layer-coated mica).

### [Example 10]

A 200 mL reaction vessel was charged with 176.3 g of water, 13.1 g of the mesoporous silica layer-coated mica (M1-2) prepared in Example 2, 0.638 g of dodecyltrimethylammonium chloride (a concentration approximately 1 time the critical micelle concentration), 16.4 g of the dispersion of the sol-gel silica (S-5) (solid concentration: 15.0% by mass) prepared in Synthesis Example 5, and 0.06 g of 29% by mass aqueous ammonia. The mixture was cooled to 5 to 10°C. Subsequently, 3.09 g of tetramethoxysilane (concentration: 90 mmol/L) was added dropwise. The reaction was performed at 5 to 10°C for 4 hours. After the reaction, the temperature of the solution was raised to 25°C. Subsequently, 11.2 g of acetic acid was added to lower the pH to about 3, and the mixture was reacted at 25°C for 16 hours. After the reaction, the precipitate was filtered, washed with water, and dried under reduced pressure for 3 hours under conditions of 100°C and -100 kPa. Thus, 13 g of a composite powder of M1-2 and S-5 was obtained (the weight of the sol-gel silica particles was 17% by mass relative to the mesoporous silica layer-coated mica).

### [Example 11]

A 200 mL reaction vessel was charged with 183.7 g of water, 11.3 g of the mesoporous silica layer-coated mica (M1-2) prepared in Example 2, 0.638 g of dodecyltrimethylammonium chloride (a concentration approximately 1 time the critical micelle concentration), 3.9 g of the dispersion of the sol-gel silica (S-2) (solid concentration: 15.0% by mass) prepared in Synthesis Example 2, and 0.06 g of 29% by mass aqueous ammonia. The mixture was cooled to 5 to 10°C. Subsequently, 3.09 g of tetramethoxysilane (concentration: 90 mmol/L) was added dropwise. The reaction was performed at 5 to 10°C for 4 hours. After the reaction, the temperature of the solution was raised to 25°C. Subsequently, 11.2 g of acetic acid was added to lower the pH to about 3, and the mixture was reacted at 25°C for 16 hours. After the reaction, the precipitate was filtered, washed with water, and dried under reduced pressure for 3 hours under conditions of 100°C and -100 kPa. Thus, 11 g of a composite powder of M1-2 and S-2 was obtained (the weight of the sol-gel silica particles was 6% by mass relative to the mesoporous silica layer-coated mica).

### [Example 12]

A 200 mL reaction vessel was charged with 170.9 g of water, 11.3 g of the mesoporous silica layer-coated mica (M1-2) prepared in Example 2, 0.638 g of dodecyltrimethylammonium chloride (a concentration approximately 1 time the critical micelle concentration), 18.5 g of the dispersion of the sol-gel silica (S-2) (solid concentration: 15.0% by mass) prepared in Synthesis Example 2, and 0.06 g of 29% by mass aqueous ammonia. The mixture was cooled to 5 to 10°C. Subsequently, 3.09 g of tetramethoxysilane (concentration: 90 mmol/L) was added dropwise. The reaction was performed at 5 to 10°C for 4 hours. After the reaction, the temperature of the solution was raised to 25°C. Subsequently, 11.2 g of acetic acid was added to lower the pH to about 3, and the mixture was reacted at 25°C for 16 hours. After the reaction, the precipitate was filtered, washed with water, and dried under reduced pressure for 3 hours under conditions of 100°C and -100 kPa. Thus, 13 g of a composite powder of M1-2 and S-2 was obtained (the weight of the sol-gel silica particles was 24% by mass relative to the mesoporous silica layer-coated mica).

### [Example 13]

A 200 mL reaction vessel was charged with 172.8 g of water, 11.3 g of the mesoporous silica layer-coated mica (M1-2) prepared in Example 2, 0.638 g of dodecyltrimethylammonium chloride (a concentration approximately 1 time the critical micelle concentration), 2.0 g of the dispersion of the sol-gel silica (S-2) (solid concentration: 15.0% by mass) prepared in Synthesis Example 2, and 0.06 g of 29% by mass aqueous ammonia. The mixture was cooled to 5 to 10°C. Subsequently, 3.09 g of tetramethoxysilane (concentration: 90 mmol/L) was added dropwise. The reaction was performed at 5 to 10°C for 4 hours. After the reaction, the temperature of the solution was raised to 25°C. Subsequently, 11.2 g of acetic acid was added to lower the pH to about 3, and the mixture was reacted at 25°C for 16 hours. After the reaction, the precipitate was filtered, washed with water, and dried under reduced pressure for 3 hours under conditions of 100°C and -100 kPa. Thus, 11 g of a composite powder of M1-2 and S-2 was obtained (the weight of the sol-gel silica particles was 3% by mass relative to the mesoporous silica layer-coated mica).

### [Example 14]

A 200 mL reaction vessel was charged with 11.3 g of talc (JA-46R, manufactured by ASADA MILLING CO., LTD.), 208.4 g of water, 0.638 g of dodecyltrimethylammonium chloride (a concentration approximately 1 time the critical micelle concentration), and 0.06 g of 29% by mass aqueous ammonia. The mixture was cooled to 5 to 10°C. Subsequently, 4.635 g of tetramethoxysilane (concentration: 135 mmol/L) was added dropwise. The mixture was reacted at 5 to 10°C for 4 hours. After the reaction, the temperature of the solution was raised to 25°C. Subsequently, 12.5 g of acetic acid was added, and the mixture was reacted at 25°C for 16 hours. After the reaction, the precipitate was filtered, washed with water, and dried under reduced pressure for 3 hours under conditions of 100°C and -100 kPa. Thus, mesoporous silica layer-coated talc (T1-2, mesoporous silica layer: 41% by mass) was obtained. A 200 mL reaction vessel was charged with 176.3 g of water, 11.3 g of the mesoporous silica layer-coated talc (T1-2), 0.638 g of dodecyltrimethylammonium chloride, 13.1 g of the dispersion of the sol-gel silica (S-2) (solid concentration: 15.0% by mass) prepared in Synthesis Example 2, and 0.06 g of 29% by mass aqueous ammonia. The mixture was cooled to 5 to 10°C. Subsequently, 3.09 g of tetramethoxysilane (concentration: 90 mmol/L) was added dropwise. The reaction was performed at 5 to 10°C for 4 hours. After the reaction, the temperature of the solution was raised to 25°C. Subsequently, 11.2 g of acetic acid was added to lower the pH to about 3, and the mixture was reacted at 25°C for 16 hours. After the reaction, the precipitate was filtered, washed with water, and dried under reduced pressure for 3 hours under conditions of 100°C and -100 kPa. Thus, 12 g of a composite powder of T1-2 and S-2 was obtained (the weight of the sol-gel silica particles was 17% by mass relative to the mesoporous silica layer-coated talc).

### [Example 15]

The mesoporous silica layer-coated mica M1-3 obtained in Example 3 was dried under atmospheric pressure at 200°C for 1 hour. Thus, mesoporous silica layer-coated mica (M1-3B, mesoporous silica layer: 59% by mass) having a pore size different from that of M1-3 was obtained.

### [Example 16]

The composite powder of M1-2 and S-2 obtained in Example 6 was dried under atmospheric pressure at 200°C for 1 hour. Thus, a composite powder of M1-2B having a pore size different from that of M1-2, and S-2 was obtained (the weight of the sol-gel silica particles was 17% by mass relative to the mesoporous silica layer-coated mica).

### [Example 17]

The composite powder of M1-2 and S-1 obtained in Example 7 was dried under atmospheric pressure at 200°C for 1 hour. Thus, a composite powder of M1-2B having a pore size different from that of M1-2, and S-1 was obtained (the weight of the sol-gel silica particles was 17% by mass relative to the mesoporous silica layer-coated mica).

### [Comparative Example 1]

A 200 mL reaction vessel was charged with 11.3 g of mica (Y-2300, manufactured by YAMAGUCHI MICA CO., LTD.), 209.0 g of water, and 0.06 g of 29% by mass aqueous ammonia. The mixture was cooled to 5 to 10°C. Subsequently, 4.635 g of tetramethoxysilane (concentration: 135 mmol/L) was added dropwise. The mixture was reacted at 5 to 10°C for 4 hours. After the reaction, the temperature of the solution was raised to 25°C. Subsequently, 12.5 g of acetic acid was added to lower the pH to about 3, and the mixture was reacted at 25°C for 16 hours. After the reaction, the precipitate was filtered, washed with water, and dried under reduced pressure for 3 hours under conditions of 100°C and -100 kPa. Non-porous silica layer-coated mica (M3-1, silica layer: 41% by mass) was thus obtained.

### [Comparative Example 2]

A 200 mL reaction vessel was charged with 176.3 g of water, 11.3 g of the non-porous silica layer-coated mica (M3-1) prepared in Comparative Example 1, 13.1 g of the dispersion of the sol-gel silica (S-2) (solid concentration: 15.0% by mass) prepared in Synthesis Example 2, and 0.06 g of 29% by mass aqueous ammonia. The mixture was cooled to 5 to 10°C. Subsequently, 3.09 g of tetramethoxysilane (concentration: 90 mmol/L) was added dropwise. The reaction was performed at 5 to 10°C for 4 hours. After the reaction, the temperature of the solution was raised to 25°C. Subsequently, 11.2 g of acetic acid was added to lower the pH to about 3, and the mixture was reacted at 25°C for 16 hours. After the reaction, the precipitate was filtered, washed with water, and dried under reduced pressure for 3 hours under conditions of 100°C and -100 kPa. Thus, 12 g of a composite powder of M3-1 and S-2 was obtained (the weight of the sol-gel silica particles was 17% by mass relative to the non-porous silica layer-coated mica).

### [Comparative Example 3]

A 100 mL reaction vessel was charged with 20 g of mica (Y-2300, manufactured by YAMAGUCHI MICA CO., LTD.) and 3.4 g of the powder (S-2-2) of the sol-gel silica particles S-2 prepared in Synthesis Example 2. The mixture was stirred at 25°C for 24 hours to give 22 g of a composite powder in which S-2-2 was electrostatically adsorbed to Y-2300 (the weight of the sol-gel silica particles was 17% by mass relative to the mica).

### [Comparative Example 4]

Mica (Y-2300, manufactured by YAMAGUCHI MICA CO., LTD.) was used.

### [Comparative Example 5]

As a spherical silica-coated plate-like powder having no mesoporous silica layer, use was made of a composite powder in which mica was coated with 10% by mass of silica having an average particle diameter of 300 nm (product name: Velvet Veil 310, manufactured by JGC Catalysts and Chemicals Ltd.).

### <Measurement of pore size>

The particles obtained in Examples 1 to 17 and Comparative Examples 1 to 5 were analyzed by the BJH method using a high-precision gas adsorption analyzer (BELSORP-mini II, manufactured by MicrotracBEL Corp.) to determine the pore size (diameter).

The pore size (diameter) of the powder was 4 nm (3.5 to 4.4 nm) in Examples 1 to 14, and was 1 nm in Examples 15 to 17. The particles in Comparative Examples had no pores. Observation with a scanning electron microscope showed that all the powders of Examples had the same aspect ratio as the plate-like powder before being coated with silica.

### <Measurement of specific surface area>

The particles obtained in Examples 1 to 17 and Comparative Examples 1 to 5 were analyzed by the single-point BET method using a high-precision gas adsorption analyzer (BELSORP MAX II, manufactured by MicrotracBEL Corp.) to determine the specific surface area. The measurement results of specific surface area are described in Table 2 below.

**[Table 2]**

| | Substrate | Particles attached | Specific surface area |
|---|---|---|---|
| Example 1 | M1-1 | - | 100 |
| Example 2 | M1-2 | - | 120 |
| Example 3 | M1-3 | - | 160 |
| Example 4 | M1-4 | - | 20 |
| Example 5 | M1-5 | - | 330 |
| Example 6 | M1-2 | S-2 | 190 |
| Example 7 | M1-2 | S-1 | 220 |
| Example 8 | M1-2 | S-3 | 150 |
| Example 9 | M1-2 | S-4 | 240 |
| Example 10 | M1-2 | S-5 | 130 |
| Example 11 | M1-2 | S-2 | 170 |
| Example 12 | M1-2 | S-2 | 220 |
| Example 13 | M1-2 | S-2 | 140 |
| Example 14 | T1-2 | S-2 | 160 |
| Example 15 | M1-3B | - | 160 |
| Example 16 | M1-2B | S-2 | 190 |
| Example 17 | M1-2B | S-1 | 220 |
| Comparative Example 1 | M3-1 | - | 4 |
| Comparative Example 2 | M3-1 | S-2 | 8 |
| Comparative Example 3 | Y-2300 | S-2-2 | 8 |
| Comparative Example 4 | Y-2300 | - | 4 |
| Comparative Example 5 | Velvet Veil 310 | | 5 |

The results in Table 2 show that a large specific surface area was obtained in Examples 1 to 17. In contrast, the powders of Comparative Examples 1 to 5 had a far smaller specific surface area than the powders of the present invention.

### <II. Formulation Examples (cosmetics) and Comparative Formulation Examples (cosmetics)>

The advantageous effects of the present invention will be described in greater detail below by presenting Formulation Examples (cosmetics) and Comparative Formulation Examples (cosmetics). However, the present invention is not limited to these Formulation Examples.

### (1) Evaluation of usability

Examples 3 and 6 to 17, and Comparative Examples 1 to 5 were applied to cosmetics, and usability was evaluated.

Water-in-oil creams of Formulation Examples 1 to 13 and Comparative Formulation Examples 1 to 5 were evaluated in terms of usability at the time of application (spreadability and uniform attachment to the skin) and post-application properties (natural finish: finish with transparency and moderate gloss, the level of concealing blemishes and wrinkles, and cosmetic durability).

Powder foundations of Formulation Examples 14 to 26 and Comparative Formulation Examples 6 to 10 were evaluated in terms of moldability, usability at the time of application (powder pick-up, spreadability, and uniform attachment to the skin), and post-application properties (the level of squeaking, and cosmetic durability).

Water-in-oil sunscreen creams involving the formulations of Formulation Examples 27 to 39 and Comparative Formulation Examples 11 to 13 were evaluated in terms of usability at the time of application (spreadability and uniform attachment to the skin) and post-application properties (the level of concealing blemishes and wrinkles, and the level of non-oiliness of the film).

The evaluation was made by a panel of 10 professional members. The items were rated according to the evaluation criteria described in Table 3, and the results of the 10 members were averaged. Each item was judged according to the judgement criteria below. The results for the water-in-oil creams are described in Table 4 below, the results for the powder foundations in Table 5 below, and the results for the water-in-oil sunscreen creams in Table 6 below.

**[Table 3]**

| Points | Ratings |
|---|---|
| 5 points | Good |
| 4 points | Rather good |
| 3 points | Fair |
| 2 points | Rather poor |
| 1 point | Poor |

### Judgment criteria for usability

⊚: The average score is 4.0 points or more.
○: The average score is 3.0 points or more and less than 4.0 points.
△: The average score is 2.0 points or more and less than 3.0 points.
×: The average score is less than 2.0 points.

The cosmetic failed the evaluation when rated × in at least one of the items.

### [Formulation Examples 1 to 13 and Comparative Formulation Examples 1 to 5: Water-in-oil creams]

| Formulation | | % by mass |
|---|---|---|
| 1. | KSG-210 (Note 1) | 3.5 |
| 2. | KSG-15 (Note 2) | 3.0 |
| 3. | KF-6017 (Note 3) | 0.5 |
| 4. | Dimethicone (6 cs) | 5.0 |
| 5. | Cyclopentasiloxane | 6.5 |
| 6. | Powder of any of Examples 3 and 6 to 17, and Comparative Examples 1 to 5 | 5.0 |
| 7. | BG | 5.5 |
| 8. | Sodium citrate | 0.2 |
| 9. | Sodium chloride | 0.5 |
| 10. | Water | 70.3 |
| Total | | 100.0 |

| | |
|---|---|
| (Note 1) | Shin-Etsu Chemical Co., Ltd.: Mixture of 70-80% by mass dimethicone + 20-30% by mass (dimethicone/(PEG-10/15)) crosspolymer |
| (Note 2) | Shin-Etsu Chemical Co., Ltd.: Mixture of 90-96% by mass cyclopentasiloxane + 4-10% by mass (dimethicone/vinyl dimethicone) crosspolymer |
| (Note 3) | Shin-Etsu Chemical Co., Ltd.: PEG-10 dimethicone |

### (Production method)

A: Components 1 to 6 were mixed uniformly.
B: Components 7 to 10 were mixed uniformly.
C: The mixture obtained in step B was added to the mixture obtained in step A. The resultant mixture was mixed uniformly.
D: The mixture obtained in step C was defoamed and then added into a container. A water-in-oil cream was thus obtained.

**[Table 4]**

| | Powder added | Usability at the time of application | | Post-application properties | | |
|---|---|---|---|---|---|---|
| | | Spreadability | Uniform attachment | Natural finish (transparency) | Level of concealing blemishes and wrinkles (correction of unevenness on the skin) | Cosmetic durability |
| Formulation Example 1 | Example 3 | Δ | ○ | ○ | ○ | ⊚ |
| Formulation Example 2 | Example 6 | ⊚ | ⊚ | ⊚ | ⊚ | ⊚ |
| Formulation Example 3 | Example 7 | ○ | ⊚ | ○ | ⊚ | ⊚ |
| Formulation Example 4 | Example 8 | ⊚ | ○ | ○ | ○ | ⊚ |
| Formulation Example 5 | Example 9 | Δ | ⊚ | ○ | ○ | ○ |
| Formulation Example 6 | Example 10 | ⊚ | Δ | ○ | ○ | ○ |
| Formulation Example 7 | Example 11 | ○ | ○ | ○ | ○ | ○ |
| Formulation Example 8 | Example 12 | ○ | ⊚ | ○ | ⊚ | ⊚ |
| Formulation Example 9 | Example 13 | ○ | Δ | ○ | Δ | ○ |
| Formulation Example 10 | Example 14 | ⊚ | ⊚ | Δ | Δ | ○ |
| Formulation Example 11 | Example 15 | Δ | ○ | ○ | ○ | ⊚ |
| Formulation Example 12 | Example 16 | ⊚ | ⊚ | ⊚ | ⊚ | ⊚ |
| Formulation Example 13 | Example 17 | ○ | ⊚ | ○ | ⊚ | ⊚ |
| Comparative Formulation Example 1 | Comparative Example 1 | Δ | Δ | Δ | × | × |
| Comparative Formulation Example 2 | Comparative Example 2 | ○ | ○ | Δ | Δ | × |
| Comparative Formulation Example 3 | Comparative Example 3 | Δ | × | × | × | × |
| Comparative Formulation Example 4 | Comparative Example 4 | Δ | Δ | Δ | × | × |
| Comparative Formulation Example 5 | Comparative Example 5 | ○ | Δ | ○ | × | × |

The results in Table 4 show that the cosmetics of Formulation Examples 1 to 13 achieved a finish with transparency and moderate gloss, and effectively corrected unevenness on the skin to which they were applied. In particular, the cosmetics of Formulation Examples 2 to 4, 7, 8, 10, 12, and 13 performed particularly well, with ○ or ⊚ rating, in spreadability at the time of application and uniform attachment to the skin. In contrast, the cosmetics of Comparative Formulation Examples 1 to 5 were poorly effective in correcting unevenness on the skin and were poor in cosmetic durability.

### [Formulation Examples 14 to 26 and Comparative Formulation Examples 6 to 10: Powder foundations]

| Formulation | | % by mass |
|---|---|---|
| 1. | Powder of any of Examples 3 and 6 to 17, and Comparative Examples 1 to 5 | 15.0 |
| 2. | Zinc stearate | 2.0 |
| 3. | AES-3083 (Note 1) treated mica | 30.0 |
| 4. | AES-3083 (Note 1) treated talc | 34.9 |
| 5. | KTP-09W, R, Y, B (Note 2) | 9.6 |
| 6. | Triethylhexanoin | 4.5 |
| 7. | Dipentaerythrityl hexa(hydroxystearate/stearate/rosinate) | 0.5 |
| 8. | KF-6038 (Note 3) | 0.5 |
| 9. | KF-56A (Note 4) | 1.0 |
| 10. | Dimethicone (100 cs) | 2.0 |
| Total | | 100.0 |

| | |
|---|---|
| (Note 1) | Shin-Etsu Chemical Co., Ltd.: Triethoxycaprylylsilane |
| (Note 2) | Shin-Etsu Chemical Co., Ltd.: KF-9909-treated coloring inorganic pigments, W: white, R: red, Y: yellow, B: black |
| (Note 3) | Shin-Etsu Chemical Co., Ltd.: Lauryl PEG-9 polydimethylsiloxyethyl dimethicone |
| (Note 4) | Shin-Etsu Chemical Co., Ltd.: Diphenylsiloxyphenyl trimethicone |

### (Production method)

A: Components 1 to 5 were mixed uniformly using a Henschel mixer.
B: Components 6 to 10 were mixed uniformly.
C: The mixture obtained in step B was added to the mixture obtained in step A. The resultant mixture was mixed uniformly using a Henschel mixer.
D: The mixture obtained in step C was passed through a sieve and was compacted in a metal dish using a mold to give a powder foundation.

**[Table 5]**

| | Powder added | Moldability | Usability at the time of application | | | Post-application properties | |
|---|---|---|---|---|---|---|---|
| | | | Powder pick-up | Spreadability | Uniform attachment | Level of squeaking | Cosmetic durability |
| Formulation Example 14 | Example 3 | ⊚ | ⊚ | Δ | ○ | Δ | ⊚ |
| Formulation Example 15 | Example 6 | ⊚ | ⊚ | ⊚ | ⊚ | ⊚ | ⊚ |
| Formulation Example 16 | Example 7 | ⊚ | ○ | O | ⊚ | ○ | ⊚ |
| Formulation Example 17 | Example 8 | ○ | ⊚ | ⊚ | ○ | ○ | ⊚ |
| Formulation Example 18 | Example 9 | ⊚ | Δ | Δ | ○ | ○ | ○ |
| Formulation Example 19 | Example 10 | Δ | ○ | ⊚ | Δ | Δ | ○ |
| Formulation Example 20 | Example 11 | ⊚ | ⊚ | O | ○ | ○ | ○ |
| Formulation Example 21 | Example 12 | ○ | ○ | ○ | ⊚ | ○ | ⊚ |
| Formulation Example 22 | Example 13 | ○ | Δ | ○ | Δ | Δ | ○ |
| Formulation Example 23 | Example 14 | ⊚ | ○ | ○ | ○ | O | ○ |
| Formulation Example 24 | Example 15 | ⊚ | ⊚ | Δ | Δ | Δ | ⊚ |
| Formulation Example 25 | Example 16 | ⊚ | ⊚ | ⊚ | ⊚ | ⊚ | ⊚ |
| Formulation Example 26 | Example 17 | ⊚ | ○ | ○ | ⊚ | ○ | ⊚ |
| Comparative Formulation Example 6 | Comparative Example 1 | Δ | Δ | Δ | Δ | × | × |
| Comparative Formulation Example 7 | Comparative Example 2 | ○ | Δ | ○ | Δ | Δ | × |
| Comparative Formulation Example 8 | Comparative Example 3 | Δ | Δ | Δ | × | × | × |
| Comparative Formulation Example 9 | Comparative Example 4 | Δ | ○ | ○ | Δ | Δ | × |
| Comparative Formulation Example 10 | Comparative Example 5 | ○ | ○ | ○ | ○ | ○ | × |

The results in Table 5 show that the cosmetics of Formulation Examples 14 to 26 had good usability and achieved cosmetic durability effects. In particular, the cosmetics of Formulation Examples 15 to 17, 20, 21, 23, 25, and 26 performed particularly well in spreadability at the time of application and uniform attachment to the skin. In contrast, the cosmetics of Comparative Formulation Examples 6 to 10 were poor in cosmetic durability effects after application. In view of the fact that powder foundations are highly loaded with powder, the level of squeaking after application was examined. The cosmetics of Formulation Examples 14 to 26 showed no problems and had good usability. In general, composite powders with a large surface area sometimes have poor press moldability or powder pick-up properties. The powders of the present invention did not have such problems and, in particular, the cosmetics of Formulation Examples 15 and 25 that contained the powders of Examples 6 and 16 performed particularly well in all the evaluation items.

### [Formulation Examples 27 to 37 and Comparative Formulation Examples 11 to 13: Water-in-oil sunscreen creams]

| Formulation | | % by mass |
|---|---|---|
| 1. | KSG-240 (Note 1) | 2.0 |
| 2. | KSG-18A (Note 2) | 2.0 |
| 3. | KF-6048 (Note 3) | 1.5 |
| 4. | Cyclopentasiloxane | 8.0 |
| 5. | Isotridecyl isononanoate | 3.0 |
| 6. | Disteardimonium hectorite | 0.8 |
| 7. | Ethylhexyl methoxycinnamate | 7.0 |
| 8. | Diethylamino hydroxybenzoyl hexyl benzoate | 2.0 |
| 9. | KF-56A (Note 4) | 5.0 |
| 10. | Powder of any of Examples 6 to 14, 16, and 17, and Comparative Examples 3 to 5 | 3.0 |
| 11. | SPD-T7 (Note 5) | 12.0 |
| 12. | SPD-Z5 (Note 6) | 12.0 |
| 13. | BG | 3.0 |
| 14. | Ethanol | 5.0 |
| 15. | Sodium citrate | 0.2 |
| 16. | Sodium chloride | 0.5 |
| 17. | Water | 34.0 |
| Total | | 100.0 |

| | |
|---|---|
| (Note 1) | Shin-Etsu Chemical Co., Ltd.: Mixture of 75-85% by mass cyclopentasiloxane + 15-25% by mass (dimethicone/(PEG-10/15)) crosspolymer |
| (Note 2) | Shin-Etsu Chemical Co., Ltd.: Mixture of 80-90% by mass diphenylsiloxyphenyl trimethicone + 10-20% by mass (dimethicone/phenyl vinyl dimethicone) crosspolymer |
| (Note 3) | Shin-Etsu Chemical Co., Ltd.: Cetyl PEG/PPG-10/1 dimethicone |
| (Note 4) | Shin-Etsu Chemical Co., Ltd.: Diphenylsiloxyphenyl trimethicone |
| (Note 5) | Shin-Etsu Chemical Co., Ltd.: Dispersion containing 45% by mass titanium oxide microparticles in cyclopentasiloxane solvent |
| (Note 6) | Shin-Etsu Chemical Co., Ltd.: Dispersion containing 60% by mass zinc oxide microparticles in cyclopentasiloxane solvent |

### (Production method)

A: Components 1 to 10 were mixed uniformly.
B: Components 13 to 17 were mixed uniformly.
C: The mixture obtained in step B was added to the mixture obtained in step A. The resultant mixture was mixed uniformly.
D: Components 11 and 12 were added to the mixture obtained in step C. The resultant mixture was mixed uniformly.
E: The mixture obtained in step D was defoamed and then added into a container. A water-in-oil sunscreen cream was thus obtained.

**[Table 6]**

| | Powder added | Usability at the time of application | | Post-application properties | |
|---|---|---|---|---|---|
| | | Spreadability | Uniform attachment | Level of concealing blemishes and wrinkles (correction of unevenness on the skin) | Level of non-oiliness of the film (cosmetic durability) |
| Formulation Example 27 | Example 6 | ⊚ | ⊚ | ⊚ | ⊚ |
| Formulation Example 28 | Example 7 | ○ | ⊚ | ○ | ⊚ |
| Formulation Example 29 | Example 8 | ⊚ | ○ | ○ | ○ |
| Formulation Example 30 | Example 9 | △ | ⊚ | ○ | ○ |
| Formulation Example 31 | Example 10 | ⊚ | △ | ○ | ○ |
| Formulation Example 32 | Example 11 | ○ | ○ | ○ | ○ |
| Formulation Example 33 | Example 12 | ○ | ⊚ | ○ | ⊚ |
| Formulation Example 34 | Example 13 | ○ | △ | ○ | ○ |
| Formulation Example 35 | Example 14 | ⊚ | ○ | △ | ○ |
| Formulation Example 36 | Example 16 | ⊚ | ⊚ | ⊚ | ⊚ |
| Formulation Example 37 | Example 17 | ○ | ⊚ | ○ | ⊚ |
| Comparative Formulation Example 11 | Comparative Example 3 | △ | × | × | × |
| Comparative Formulation Example 12 | Comparative Example 4 | △ | △ | × | × |
| Comparative Formulation Example 13 | Comparative Example 5 | ○ | △ | △ | × |

The results in Table 6 show that the cosmetics of Formulation Examples 27 to 37 effectively corrected unevenness on the skin to which they were applied, and effectively maintained non-oiliness on the film. In particular, the cosmetics of Formulation Examples 27 to 29, 32, 33, and 35 to 37 performed particularly well in spreadability at the time of application and uniform attachment to the skin. In contrast, the cosmetics of Comparative Formulation Examples 11 to 13 were poorly effective in correcting unevenness on the skin to which they were applied, and were poorly effective in maintaining non-oiliness on the film.

### [Formulation Example 38: Aqueous gel]

| | Formulation | % by mass |
|---|---|---|
| 1. | Composite powder obtained in Example 8 | 5.0 |
| 2. | KF-6100 (Note 1) | 0.5 |
| 3. | Ethanol | 3.0 |
| 4. | BG | 4.0 |
| 5. | Glycerin | 2.0 |
| 6. | (Ammonium acryloyldimethyltaurate/VP) copolymer | 0.2 |
| 7. | Xanthan gum | 0.2 |
| 8. | Arginine | 0.5 |
| 9. | Preservative | q.s. |
| 10. | Water | balance |
| | Total | 100.0 |

| | | |
|---|---|---|
| (Note 1) Shin-Etsu Chemical Co., Ltd.: Polyglyceryl-3 disiloxane dimethicone | | |

### (Production method)

A: Components 1 to 3 were mixed uniformly.
B: Components 4 to 10 were mixed uniformly.
C: The mixture obtained in step A was added to the mixture obtained in step B. The resultant mixture was mixed uniformly.
D: The mixture obtained in step C was defoamed and then added into a container. An aqueous gel was thus obtained.

The aqueous gel obtained as described above gave a highly dewy feeling on application, spread lightly without stickiness, had excellent adhesion, was well-fitting, and gave a matte finish with moderate luster.

### [Formulation Example 39: Oil-based gel]

| | Formulation | % by mass |
|---|---|---|
| 1. | Composite powder obtained in Example 12 | 10.0 |
| 2. | KSG-19 (Note 1) | 20.0 |
| 3. | KSG-15 (Note 2) | 30.0 |
| 4. | KF-56A (Note 3) | 5.0 |
| 5. | Dimethicone (1.5 cs) | balance |
| | Total | 100.0 |

| | |
|---|---|
| (Note 1) | Shin-Etsu Chemical Co., Ltd.: Mixture of 80-90% by mass dimethicone + 10-20% by mass (dimethicone/vinyl dimethicone) crosspolymer |
| (Note 2) | Shin-Etsu Chemical Co., Ltd.: Mixture of 90-96% by mass cyclopentasiloxane + 4-10% by mass (dimethicone/vinyl dimethicone) crosspolymer |
| (Note 3) | Shin-Etsu Chemical Co., Ltd.: Diphenylsiloxyphenyl trimethicone |

### (Production method)

A: Components 1 to 5 were mixed uniformly.
B: The mixture obtained in step A was defoamed and then added into a container. An oil-based gel was thus obtained.

The oil-based gel obtained as described above showed full smoothness on application, spread lightly without stickiness, had excellent adhesion, was well-fitting, and gave a matte finish with moderate luster.

### [Formulation Example 40: Water-in-oil cream]

| | Formulation | % by mass |
|---|---|---|
| 1. | KSG-310 (Note 1) | 3.0 |
| 2. | KSG-44 (Note 2) | 1.0 |
| 3. | KF-6048 (Note 3) | 0.2 |
| 4. | Squalane | 10.8 |
| 5. | Composite powder obtained in Example 9 | 1.0 |
| 6. | BG | 8.0 |
| 7. | Ethanol | 5.0 |
| 8. | Magnesium sulfate | 0.2 |
| 9. | Sodium chloride | 0.5 |
| 10. | Water | balance |
| | Total | 100.0 |

| | |
|---|---|
| (Note 1) | Shin-Etsu Chemical Co., Ltd.: Mixture of 65-75% by mass mineral oil + 25-35% by mass (PEG-15/lauryl dimethicone) crosspolymer |
| (Note 2) | Shin-Etsu Chemical Co., Ltd.: Mixture of 65-75% by mass squalane + 25-35% by mass (vinyl dimethicone/lauryl dimethicone) crosspolymer |
| (Note 3) | Shin-Etsu Chemical Co., Ltd.: Cetyl PEG/PPG-10/1 dimethicone |

### (Production method)

A: Components 1 to 5 were mixed uniformly.
B: Components 6 to 10 were mixed uniformly.
C: The mixture obtained in step B was added to the mixture obtained in step A. The resultant mixture was mixed uniformly.
D: The mixture obtained in step C was defoamed and then added into a container. A water-in-oil cream was thus obtained.

The water-in-oil cream of the present invention obtained as described above showed full smoothness on application, spread lightly without stickiness, had excellent adhesion, was well-fitting, and gave a natural finish with moderate oily luster.

### [Formulation Example 41: Water-in-oil cream]

| | Formulation | % by mass |
|---|---|---|
| 1. | Dimethicone (6 cs) | 6.0 |
| 2. | KF-54 (Note 1) | 4.0 |
| 3. | Neopentyl glycol dioctanoate | 3.0 |
| 4. | KF-6012 (Note 2) | 3.0 |
| 5. | Composite powder obtained in Example 14 | 3.0 |
| 6. | Glycerin | 10.0 |
| 7. | Preservative | q.s. |
| 8. | Essential oil | q.s. |
| 9. | Water | balance |
| | Total | 100.0 |

| | |
|---|---|
| (Note 1) | Shin-Etsu Chemical Co., Ltd.: Diphenyl dimethicone |
| (Note 2) | Shin-Etsu Chemical Co., Ltd.: PEG/PPG-20/22 butyl ether dimethicone |

### (Production method)

A: Components 1 to 5 were mixed uniformly.
B: Components 6, 7, and 9 were mixed to give a solution.
C: The solution obtained in step B was added to the mixture obtained in step A. The resultant mixture was mixed uniformly.
D: Component 8 was added to the mixture obtained in step C. The resultant mixture was mixed uniformly.
E: The mixture obtained in step D was defoamed and then added into a container. A water-in-oil cream was thus obtained.

The water-in-oil cream obtained as described above had a fine texture, spread lightly without stickiness or oiliness, and was free from changes due to temperature or aging, thus being shown to have excellent stability.

### [Formulation Example 42: Water-in-oil cream]

| | Formulation | % by mass |
|---|---|---|
| 1. | KSG-340 (Note 1) | 6.0 |
| 2. | Mineral oil | 8.5 |
| 3. | Macadamia nut oil | 5.0 |
| 4. | KF-6105 (Note 2) | 0.5 |
| 5. | KSP-101 (Note 3) | 3.0 |
| 6. | Composite powder obtained in Example 12 | 4.0 |
| 7. | Sodium citrate | 0.2 |
| 8. | PG | 8.0 |
| 9. | Glycerin | 3.0 |
| 10. | Preservative | q.s. |
| 11. | Fragrance | q.s. |
| 12. | Water | balance |
| | Total | 100.0 |

| | |
|---|---|
| (Note 1) | Shin-Etsu Chemical Co., Ltd.: Mixture of 65-75% by mass squalane + 25-35% by mass (PEG-10/lauryl dimethicone) crosspolymer and (PEG-15/lauryl dimethicone) crosspolymer |
| (Note 2) | Shin-Etsu Chemical Co., Ltd.: Lauryl polyglyceryl-3 polydimethylsiloxyethyl dimethicone |
| (Note 3) | Shin-Etsu Chemical Co., Ltd.: (Vinyl dimethicone/methicone silsesquioxane) crosspolymer |

### (Production method)

A: Components 1 to 6 were mixed.
B: Components 7 to 10 and 12 were mixed to give a solution.
C: The solution obtained in step B was added to the mixture obtained in step A. The resultant mixture was mixed uniformly.
D: Component 11 was added to the mixture obtained in step C. The resultant mixture was mixed uniformly.
E: The mixture obtained in step D was defoamed and then added into a container. A water-in-oil cream was thus obtained.

The water-in-oil cream obtained as described above had a fine texture, spread lightly without stickiness or oiliness, and was free from changes due to temperature or aging, thus being shown to have excellent stability.

### [Formulation Example 43: Water-in-oil cream]

| Formulation | | % by mass |
|---|---|---|
| 1. | Cyclopentasiloxane | 10.5 |
| 2. | Dimethicone (6 cs) | 4.0 |
| 3. | KF-6028 (Note 1) | 3.0 |
| 4. | Octyldodeceth-5 | 1.0 |
| 5. | Polysorbate 60 | 0.5 |
| 6. | Composite powder obtained in Example 10 | 15.0 |
| 7. | Mineral oil | 2.0 |
| 8. | Macadamia nut oil | 1.0 |
| 9. | Scutellaria root extract (Note 2) | 1.0 |
| 10. | Gentian extract (Note 3) | 0.5 |
| 11. | Ethanol | 3.0 |
| 12. | BG | 4.0 |
| 13. | Preservative | q.s. |
| 14. | Water | balance |
| Total | | 100.0 |

| | |
|---|---|
| (Note 1 ) | Shin-Etsu Chemical Co., Ltd.: PEG-9 polydimethylsiloxyethyl dimethicone |
| (Note 2 ) | Scutellaria root extract: 50% by mass, extracted with BG water |
| (Note 3 ) | Gentian extract: 20% by mass, extracted with ethanol water |

### (Production method)

A: Components 1 to 8 were mixed.
B: Components 9 to 14 were mixed to give a solution.
C: The solution obtained in step B was added to the mixture obtained in step A. The resultant mixture was mixed uniformly.
D: The mixture obtained in step C was defoamed and then added into a container. A water-in-oil cream was thus obtained.

The water-in-oil cream obtained as described above had a fine texture, was free of stickiness, spread lightly, had excellent adhesion, and was very long-lasting. Furthermore, the water-in-oil cream was free from changes due to temperature or aging, thus being shown to have excellent stability.

### [Formulation Example 44: Oil-in-water cream]

| Formulation | | % by mass |
|---|---|---|
| 1. | KSG-43 (Note 1) | 5.0 |
| 2. | Triethylhexanoin | 5.0 |
| 3. | Composite powder obtained in Example 11 | 11.0 |
| 4. | DPG | 7.0 |
| 5. | Glycerin | 5.0 |
| 6. | METOLOSE SM-4000 (Note 2) (2% aqueous solution) | 7.0 |
| 7. | Polyacrylamide emulsifier (Note 3) | 2.0 |
| 8. | Preservative | q.s. |
| 9. | Fragrance | q.s. |
| 10. | Water | balance |
| Total | | 100.0 |

| | |
|---|---|
| (Note 1) | Shin-Etsu Chemical Co., Ltd.: Mixture of 65-75% by mass triethylhexanoin + 25-35% by mass (vinyl dimethicone/lauryl dimethicone) crosspolymer |
| (Note 2) | Shin-Etsu Chemical Co., Ltd.: Methylcellulose |
| (Note 3) | SEPPIC: SEPIGEL 305 |

### (Production method)

A: Components 1 to 3 were mixed.
B: Components 4 to 10 were mixed.
C: The mixture obtained in step B was added to the mixture obtained in step A. The resultant mixture was mixed uniformly.
D: The mixture obtained in step C was defoamed and then added into a container. An oil-in-water cream was thus obtained.

The oil-in-water cream obtained as described above had a fine texture, spread lightly without stickiness or oiliness, and was free from changes due to temperature or aging, thus being shown to have excellent stability.

### [Formulation Example 45: Water-in-oil sunscreen milky lotion]

| Formulation | | % by mass |
|---|---|---|
| 1. | KSG-270 (Note 1) | 3.5 |
| 2. | KSG-18A (Note 2) | 3.0 |
| 3. | KF-6048 (Note 3) | 0.5 |
| 4. | KF-56A (Note 4) | 5.0 |
| 5. | KF-4418 (Note 5) | 7.5 |
| 6. | KF-4422 (Note 6) | 6.5 |
| 7. | Ethylhexyl triazone | 5.0 |
| 8. | Ethylhexyl salicylate | 2.0 |
| 9. | Octocrylene | 3.0 |
| 10. | Bis-ethylhexyloxyphenol methoxyphenyltriazine | 1.0 |
| 11. | Composite powder obtained in Example 13 | 4.0 |
| 12. | BG | 5.5 |
| 13. | Sodium citrate | 0.2 |
| 14. | Sodium chloride | 0.5 |
| 15. | Water | balance |
| Total | | 100.0 |

| | |
|---|---|
| (Note 1) | Shin-Etsu Chemical Co., Ltd.: Mixture of 75-85% by mass diphenylsiloxyphenyl trimethicone + 15-25% by mass (dimethicone/(PEG-10/15)) crosspolymer |
| (Note 2) | Shin-Etsu Chemical Co., Ltd.: Mixture of 80-90% by mass diphenylsiloxyphenyl trimethicone + 10-20% by mass (dimethicone/phenyl vinyl dimethicone) crosspolymer |
| (Note 3) | Shin-Etsu Chemical Co., Ltd.: Cetyl PEG/PPG-10/1 dimethicone |
| (Note 4) | Shin-Etsu Chemical Co., Ltd.: Diphenylsiloxyphenyl trimethicone |
| (Note 5) | Shin-Etsu Chemical Co., Ltd.: Caprylyl methicone |
| (Note 6) | Shin-Etsu Chemical Co., Ltd.: Ethyl trimethicone |

### (Production method)

A: Components 1 to 11 were mixed uniformly.
B: Components 12 to 15 were mixed uniformly.
C: The mixture obtained in step B was added to the mixture obtained in step A. The resultant mixture was mixed uniformly.
D: The mixture obtained in step C was defoamed and then added into a container. A water-in-oil sunscreen milky lotion was thus obtained.

The water-in-oil sunscreen milky lotion obtained as described above showed full smoothness on application, gave a finish with moderate luster, and was long-lasting, thus showing high usefulness.

### [Formulation Example 46: Water-in-oil sunscreen cream]

| Formulation | | % by mass |
|---|---|---|
| 1. | KSG-270 (Note 1) | 5.0 |
| 2. | KF-6105 (Note 2) | 2.5 |
| 3. | Cyclopentasiloxane | 11.5 |
| 4. | Ethylhexyl palmitate | 5.0 |
| 5. | Ethylhexyl methoxycinnamate | 7.0 |
| 6. | Homosalate | 3.0 |
| 7. | KP-550 (Note 3) | 12.0 |
| 8. | Antioxidant | q.s. |
| 9. | Composite powder obtained in Example 9 | 8.0 |
| 10. | AES-3083 (Note 4) treated zinc oxide microparticles | 15.0 |
| 11. | Sodium chloride | 0.5 |
| 12. | BG | 2.0 |
| 13. | Preservative | q.s. |
| 14. | Fragrance | q.s. |
| 15. | Water | balance |
| Total | | 100.0 |

| | |
|---|---|
| (Note 1) | Shin-Etsu Chemical Co., Ltd.: Mixture of 75-85% by mass diphenylsiloxyphenyl trimethicone + 15-25% by mass (dimethicone/(PEG-10/15)) crosspolymer |
| (Note 2) | Shin-Etsu Chemical Co., Ltd.: Lauryl polyglyceryl-3 polydimethylsiloxyethyl dimethicone |
| (Note 3) | Shin-Etsu Chemical Co., Ltd.: Solution of 60% by mass isododecane + 40% (acrylates/dimethicone) copolymer |
| (Note 4) | Shin-Etsu Chemical Co., Ltd.: Triethoxycaprylylsilane |

### (Production method)

A: Component 3 was added to a portion of component 2 and mixed uniformly. Component 10 was added. The resultant mixture was dispersed using a bead mill.
B: The remainder of component 2, component 1, and components 4 to 9 were mixed uniformly.
C: Components 11 to 13 and 15 were mixed to give a solution.
D: The solution obtained in step C was added to the mixture obtained in step B. The resultant mixture was mixed uniformly.
E: The mixture obtained in step A was added to the mixture obtained in step D. The resultant mixture was mixed uniformly.
F: Component 12 was added to the mixture obtained in step E. The resultant mixture as mixed uniformly.
G: The mixture obtained in step F was defoamed and then added into a container. A water-in-oil sunscreen cream was thus obtained.

The water-in-oil sunscreen cream obtained as described above was free of stickiness, spread lightly, had excellent adhesion, effectively corrected morphological problems on the skin, and was very long-lasting. Furthermore, the water-in-oil sunscreen cream was very stable to changes due to temperature or aging.

### [Formulation Example 47: Water-in-oil sunscreen milky lotion]

| Formulation | | % by mass |
|---|---|---|
| 1. | Cyclopentasiloxane | 20.0 |
| 2. | KF-6038 (Note 1) | 0.5 |
| 3. | KF-56A (Note 2) | 3.0 |
| 4. | Sorbitan isostearate | 1.0 |
| 5. | X-21-5250 (Note 3) | 1.0 |
| 6. | Composite powder obtained in Example 8 | 6.0 |
| 7. | KSP-105 (Note 4) | 2.0 |
| 8. | Isostearic acid-treated titanium oxide microparticles | 10.0 |
| 9. | Isostearic acid-treated zinc oxide microparticles | 10.0 |
| 10. | Sorbitol | 2.0 |
| 11. | Sodium chloride | 2.0 |
| 12. | Preservative | q.s. |
| 13. | Fragrance | q.s. |
| 14. | Water | balance |
| Total | | 100.0 |

| | |
|---|---|
| (Note 1) | Shin-Etsu Chemical Co., Ltd.: Lauryl PEG-9 polydimethylsiloxyethyl dimethicone |
| (Note 2) | Shin-Etsu Chemical Co., Ltd.: Diphenylsiloxyphenyl trimethicone |
| (Note 3) | Shin-Etsu Chemical Co., Ltd.: Solution of 50% by mass cyclopentasiloxane + 50% trimethylsiloxysilicate |
| (Note 4) | Shin-Etsu Chemical Co., Ltd.: (Vinyl dimethicone/methicone silsesquioxane) crosspolymer |

### (Production method)

A: Component 2 was added to component 1 and mixed uniformly. Components 8 and 9 were added. The resultant mixture was dispersed using a bead mill.
B: Components 3 to 7 were mixed uniformly.
C: Components 10 to 12 and component 14 were mixed to give a solution.
D: The solution obtained in step C was added to the mixture obtained in step B. The resultant mixture was mixed uniformly.
E: The mixture obtained in step A was added to the mixture obtained in step D. The resultant mixture was mixed uniformly.
F: Component 13 was added to the mixture obtained in step E. The resultant mixture was mixed uniformly.
G: The mixture obtained in step F was defoamed and then added into a container. A water-in-oil sunscreen milky lotion was thus obtained.

The water-in-oil sunscreen milky lotion obtained as described above had a fine texture, spread lightly, and was free of stickiness. Furthermore, the water-in-oil sunscreen milky lotion had good cosmetic durability to offer persistent UV protection effects, and was free from changes due to temperature or aging, thus being shown to have excellent stability.

### [Formulation Example 48: Water-in-oil cream foundation]

| Formulation | | % by mass |
|---|---|---|
| 1. | KSG-710 (Note 1) | 4.0 |
| 2. | KSG-016F (Note 2) | 2.0 |
| 3. | KF-6104 (Note 3) | 3.0 |
| 4. | Dimethicone (2 cs) | 12.0 |
| 5. | Disteardimonium hectorite | 1.2 |
| 6. | Composite powder obtained in Example 10 | 2.0 |
| 7. | KF-7312L (Note 4) | 5.0 |
| 8. | Isotridecyl isononanoate | 2.0 |
| 9. | KF-6106 (Note 5) | 0.5 |
| 10. | KF-99P (Note 6) treated inorganic coloring pigment | 10.0 |
| 11. | Pentylene glycol | 5.0 |
| 12. | Sodium citrate | 0.2 |
| 13. | Sodium chloride | 0.5 |
| 14. | Fragrance | q.s. |
| 15. | Water | balance |
| Total | | 100.0 |

| | |
|---|---|
| (Note 1) | Shin-Etsu Chemical Co., Ltd.: Mixture of 70-80% by mass dimethicone + 20-30% by mass (dimethicone/polyglycerin-3) crosspolymer |
| (Note 2) | Shin-Etsu Chemical Co., Ltd.: Mixture of 70-80% by mass dimethicone + 20-30% by mass (dimethicone/vinyl dimethicone) crosspolymer |
| (Note 3) | Shin-Etsu Chemical Co., Ltd.: Polyglyceryl-3 polydimethylsiloxyethyl dimethicone |
| (Note 4) | Shin-Etsu Chemical Co., Ltd.: Solution of 50% by mass dimethicone (2 cs) + 50% trimethylsiloxysilicate |
| (Note 5) | Shin-Etsu Chemical Co., Ltd.: Polyglyceryl-3 polydimethylsiloxyethyl dimethicone |
| (Note 6) | Shin-Etsu Chemical Co., Ltd.: Methicone |

### (Production method)

A: Components 1 to 7 were mixed.
B: Components 8 to 10 were mixed and roll-treated.
C: The mixture obtained in step B was added to the mixture obtained in step A. The resultant mixture was mixed uniformly.
D: Components 11 to 13 and 15 were mixed to give a solution.
E: The solution obtained in step D was added to the mixture obtained in step C. The resultant mixture was mixed uniformly.
F: Component 14 was added to the mixture obtained in step E. The resultant mixture was mixed uniformly.
G: The mixture obtained in step F was defoamed and then added into a container. A water-in-oil cream foundation was thus obtained.

The water-in-oil cream foundation obtained as described above showed full smoothness on application and excellent moisture retaining properties, spread lightly without stickiness, had excellent adhesion, was well-fitting, and gave a natural finish with moderate luster.

### [Formulation Example 49: Water-in-oil cream foundation]

| Formulation | | % by mass |
|---|---|---|
| 1. | Cyclopentasiloxane | 45.0 |
| 2. | Dimethicone (6 cs) | 10.0 |
| 3. | KF-6028P (Note 1) | 3.5 |
| 4. | Disteardimonium hectorite | 1.5 |
| 5. | Composite powder obtained in Example 13 | 14.5 |
| 6. | Ethylhexyl palmitate | 5.0 |
| 7. | KP-578 (Note 2) | 0.4 |
| 8. | Magnesium stearate-treated inorganic coloring pigment | 8.6 |
| 9. | DPG | 5.0 |
| 10. | Preservative | q.s. |
| 11. | Essential oil | q.s. |
| 12. | Water | balance |
| Total | | 100.0 |

| | |
|---|---|
| (Note 1) | Shin-Etsu Chemical Co., Ltd.: PEG-9 polydimethylsiloxyethyl dimethicone |
| (Note 2) | Shin-Etsu Chemical Co., Ltd.: (Acrylates/ethylhexyl acrylate/dimethicone methacrylate) copolymer |

### (Production method)

A: Components 1 to 5 were mixed.
B: Components 6 to 8 were mixed and roll-treated.
C: The mixture obtained in step B was added to the mixture obtained in step A. The resultant mixture was mixed uniformly.
D: Components 9, 10, and 12 were mixed to give a solution.
E: The solution obtained in step D was added to the mixture obtained in step C. The resultant mixture was mixed uniformly.
F: Component 11 was added to the mixture obtained in step E. The resultant mixture was mixed uniformly.
G: The mixture obtained in step F was defoamed and then added into a container. A water-in-oil cream foundation was thus obtained.

The water-in-oil cream foundation obtained as described above had a fine texture, spread lightly without stickiness or oiliness, was long-lasting, and was free from changes due to temperature or aging, thus being shown to have excellent stability.

### [Formulation Example 50: Water-in-oil liquid foundation]

| Formulation | | % by mass |
|---|---|---|
| 1. | Cyclopentasiloxane | 10.0 |
| 2. | KF-56A (Note 1) | 4.5 |
| 3. | Ethylhexyl methoxycinnamate | 5.0 |
| 4. | KF-9021 (Note 2) | 1.0 |
| 5. | KF-6105 (Note 3) | 1.0 |
| 6. | Polyglyceryl-2 Isostearate | 0.5 |
| 7. | Composite powder obtained in Example 11 | 8.0 |
| 8. | Neopentyl glycol dioctanoate | 3.0 |
| 9. | Polyhydroxystearic acid | 1.0 |
| 10. | Dimethicone-treated inorganic coloring pigment | 7.0 |
| 11. | Glycerin | 3.0 |
| 12. | Preservative | q.s. |
| 13. | Water | balance |
| Total | | 100.0 |

| | |
|---|---|
| (Note 1) | Shin-Etsu Chemical Co., Ltd.: Diphenylsiloxyphenyl trimethicone |
| (Note 2) | Shin-Etsu Chemical Co., Ltd.: Solution of 50% by mass cyclopentasiloxane + 50% by mass trimethylsiloxysilicate |
| (Note 3) | Shin-Etsu Chemical Co., Ltd.: Lauryl polyglyceryl-3 polydimethylsiloxyethyl dimethicone |

### (Production method)

A: Components 1 to 7 were mixed.
B: Components 8 to 10 were mixed and roll-treated.
C: The mixture obtained in step B was added to the mixture obtained in step A. The resultant mixture was mixed uniformly.
D: Components 11 to 13 were mixed to give a solution.
E: The solution obtained in step D was added to the mixture obtained in step C. The resultant mixture was mixed uniformly.
F: The mixture obtained in step E was defoamed and then added into a container. A water-in-oil liquid foundation was thus obtained.

The water-in-oil liquid foundation obtained as described above had a low viscosity and a fine texture, spread lightly without stickiness or oiliness, effectively corrected morphological problems on the skin, was long-lasting, and was free from changes due to temperature or aging, thus being shown to have excellent stability.

### [Formulation Example 51: Water-in-oil liquid foundation]

| Formulation | | % by mass |
|---|---|---|
| 1. | Cyclopentasiloxane | 15.0 |
| 2. | KF-54 (Note 1) | 3.0 |
| 4. | KF-6017 (Note 2) | 1.5 |
| 5. | Polyglyceryl-2 diisostearate | 1.0 |
| 6. | Composite powder obtained in Example 6 | 15.0 |
| 7. | KMP-592 (Note 3) | 3.0 |
| 8. | Triethylhexanoin | 10.0 |
| 9. | KF-6115 (Note 4) | 1.0 |
| 10. | Stearic acid-treated titanium oxide microparticles | 6.0 |
| 11. | KF-9901 (Note 5) treated inorganic coloring pigment | 9.0 |
| 12. | BG | 7.0 |
| 13. | Sodium chloride | 0.5 |
| 14. | Preservative | q.s. |
| 15. | Water | balance |
| Total | | 100.0 |

| | |
|---|---|
| (Note 1) | Shin-Etsu Chemical Co., Ltd.: Diphenyl dimethicone |
| (Note 2) | Shin-Etsu Chemical Co., Ltd.: PEG-10 dimethicone |
| (Note 3) | Shin-Etsu Chemical Co., Ltd.: (Methyl/phenyl) polysilsesquioxane |
| (Note 4) | Shin-Etsu Chemical Co., Ltd.: Lauryl polyglyceryl-3 polydimethylsiloxyethyl dimethicone |
| (Note 5) | Shin-Etsu Chemical Co., Ltd.: Hydrogen dimethicone |

### (Production method)

A: Components 1 to 7 were mixed.
B: Components 8 to 11 were mixed and roll-treated.
C: The mixture obtained in step B was added to the mixture obtained in step A. The resultant mixture was mixed uniformly.
D: Components 12 to 15 were mixed to give a solution.
E: The solution obtained in step D was added to the mixture obtained in step C. The resultant mixture was mixed uniformly.
F: The mixture obtained in step E was defoamed and then added into a container. A water-in-oil liquid foundation was thus obtained.

The water-in-oil liquid foundation obtained as described above was free of stickiness, spread lightly, had excellent adhesion, and was very long-lasting. Furthermore, the water-in-oil liquid foundation was free from changes due to temperature or aging, thus being shown to have excellent stability.

### [Formulation Example 52: Oil-in-water base cream]

| Formulation | | % by mass |
|---|---|---|
| 1. | Water | balance |
| 2. | Glycerin | 3.0 |
| 3. | Xanthan gum | 0.2 |
| 4. | Pentylene glycol | 2.0 |
| 5. | Sucrose cocoate | 0.2 |
| 6. | Sorbitan stearate | 3.0 |
| 7. | PEG-60 glyceryl isostearate | 0.5 |
| 8. | Microcrystalline wax | 0.3 |
| 9. | Behenyl alcohol | 0.5 |
| 10. | Isononyl isononanoate | 6.0 |
| 11. | Composite powder obtained in Example 1 | 3.0 |
| 12. | BG | 5.0 |
| 13. | Silica-treated inorganic coloring pigment | 5.0 |
| 14. | Polysorbate 60 | 0.3 |
| 15. | (Hydroxvethyl acrylate/sodium acrvlovldimethyltaurate) copolymer | 0.6 |
| Total | | 100.0 |

### (Production method)

A: Components 1 to 7 were heated to 80°C and mixed uniformly.
B: Components 8 to 10 were heated to 80°C, and component 11 was added. The resultant mixture was mixed uniformly.
C: The hot mixture obtained in step B was added to the hot mixture obtained in step A. The resultant mixture was mixed uniformly.
D: Components 12 and 13 were mixed and roll-treated.
E: The mixture obtained in step C was cooled to room temperature, and components 14 and 15 were added. The resultant mixture was mixed uniformly.
F: The mixture obtained in step D was added to the mixture obtained in step E. The resultant mixture was mixed uniformly.
G: The mixture obtained in step F was defoamed and then added into a container. An oil-in-water base cream was thus obtained.

The oil-in-water base cream obtained as described above gave a highly dewy feeling on application, spread lightly without stickiness, had excellent adhesion, was well-fitting, and gave a matte finish with moderate luster.

### [Formulation Example 53: Oil-based cream foundation]

| Formulation | | % by mass |
|---|---|---|
| 1. | Isododecane | balance |
| 2. | KSG-42A (Note 1) | 10.0 |
| 3. | KF-6104 (Note 2) | 4.0 |
| 4. | TSPL-30-ID (Note 3) | 2.0 |
| 5. | Dimethicone (6 cs) | 5.0 |
| 6. | Ethanol | 8.0 |
| 7. | Disteardimonium hectorite | 1.5 |
| 8. | Silylated silica | 1.0 |
| 9. | Composite powder obtained in Example 2 | 6.0 |
| 10. | Isotridecyl isononanoate | 10.0 |
| 11. | KP-578 (Note 4) | 0.5 |
| 12. | KF-9901 (Note 5) treated titanium oxide microparticles | 8.0 |
| 13. | KF-9901 (Note 5) treated zinc oxide microparticles | 5.0 |
| 14. | AES-3083 (Note 6) treated inorganic coloring pigment | 7.5 |
| Total | | 100.0 |

| | |
|---|---|
| (Note 1) | Shin-Etsu Chemical Co., Ltd.: Mixture of 75-85% by mass isododecane + 15-25% by mass (vinyl dimethicone/lauryl dimethicone) crosspolymer |
| (Note 2) | Shin-Etsu Chemical Co., Ltd.: Polyglyceryl-3 polydimethylsiloxyethyl dimethicone |
| (Note 3) | Shin-Etsu Chemical Co., Ltd.: Solution of 30% by mass pullulan tri(trimethylsiloxy)silylpropyl carbamide + 70% by mass isododecane |
| (Note 4) | Shin-Etsu Chemical Co., Ltd.: (Acrylates/ethylhexyl acrylate/dimethicone methacrylate) copolymer |
| (Note 5) | Shin-Etsu Chemical Co., Ltd.: Hydrogen dimethicone |
| (Note 6) | Shin-Etsu Chemical Co., Ltd.: Triethoxycaprylylsilane |

### (Production method)

A: Components 1 to 9 were mixed uniformly.
B: Components 10 to 14 were mixed uniformly and roll-treated.
C: The mixture obtained in step B was added to the mixture obtained in step A. The resultant mixture was mixed uniformly.
D: The mixture obtained in step C was defoamed and then added into a container. An oil-based cream foundation was thus obtained.

The oil-based cream foundation obtained as described above spread lightly, was well-fitting to the skin, gave a moist finish, and formed a long-lasting firm cosmetic film with moderate luster, thus showing high usefulness.

### [Formulation Example 54: Oil-based mousse foundation]

| Formulation | | % by mass |
|---|---|---|
| 1. | Dimethicone (6 cs) | balance |
| 2. | KF-7312L (Note 1) | 10.0 |
| 3. | KSG-048Z (Note 2) | 30.0 |
| 4. | Squalane | 1.0 |
| 5. | Jojoba oil | 1.0 |
| 6. | KF-56A (Note 3) | 1.0 |
| 7. | Composite powder obtained in Example 8 | 18.0 |
| 8. | Methyl methacrylate crosspolymer | 1.0 |
| 9. | Nylon-12 | 1.0 |
| 10. | KTP-09W, R, Y, B (Note 4) | 10.0 |
| 11. | Stearic acid-treated titanium oxide microparticles | 10.0 |
| 12. | Antioxidant | q.s. |
| 13. | Dimethicone-treated talc | 3.0 |
| 14. | Dimethicone-treated mica | 5.0 |
| Total | | 100.0 |

| | |
|---|---|
| (Note 1) | Shin-Etsu Chemical Co., Ltd.: Solution of 50% by mass dimethicone (2 cs) + 50% trimethylsiloxysilicate |
| (Note 2) | Shin-Etsu Chemical Co., Ltd.: Mixture of 75-85% by mass dimethicone + 15-25% by mass (lauryl polydimethylsiloxyethyl dimethicone/bis-vinyl dimethicone) crosspolymer |
| (Note 3) | Shin-Etsu Chemical Co., Ltd.: Diphenylsiloxyphenyl trimethicone |
| (Note 4) | Shin-Etsu Chemical Co., Ltd.: KF-9909-treated coloring inorganic pigments, W: white, R: red, Y: yellow, B: black |

### (Production method)

A: A portion of component 1, and components 2 to 9 were mixed uniformly.
B: Components 10 to 14 were mixed with the remainder of component 1. The resultant mixture was roll-treated.
C: The mixture obtained in step B was added to the mixture obtained in step A. The resultant mixture was mixed uniformly.
D: The mixture obtained in step C was defoamed and then added into a container. An oil-based mousse foundation was thus obtained.

The oil-based mousse foundation obtained as described above was in a souffle-like form, was easy to pick up, spread lightly, and showed a non-oily and non-powdery feeling on use. Furthermore, the oil-based mousse foundation was long-lasting with good water resistance, water repellency, and sweat resistance, was resistant to coming off, and was free from changes due to temperature or aging, thus being shown to have excellent stability.

### [Formulation Example 55: Oil-based solid foundation]

| Formulation | | % by mass |
|---|---|---|
| 1. | Synthetic wax | 4.0 |
| 2. | Carnauba wax | 2.0 |
| 3. | Shea butter | 0.5 |
| 4. | Glyceryl tri(caprylate/caprate) | 3.0 |
| 5. | KF-56A (Note 1) | 5.0 |
| 6. | Ethylhexyl methoxycinnamate | 7.0 |
| 7. | Bis-ethylhexyloxyphenol methoxyphenyltriazine | 0.5 |
| 8. | Dimethicone (2 cs) | 5.0 |
| 9. | Isotridecyl isononanoate | balance |
| 10. | Composite powder obtained in Example 5 | 4.0 |
| 11. | KMP-591 (Note 2) | 1.0 |
| 12. | Cetyl ethylhexanoate | 9.0 |
| 13. | KF-6115 (Note 3) | 1.0 |
| 14. | KF-9901 (Note 4) treated titanium oxide microparticles | 7.0 |
| 15. | KTP-09W, R. Y. B (Note 5) | 11.0 |
| Total | | 100.0 |

| | |
|---|---|
| (Note 1) | Shin-Etsu Chemical Co., Ltd.: Diphenylsiloxyphenyl trimethicone |
| (Note 2) | Shin-Etsu Chemical Co., Ltd.: Polymethylsilsesquioxane |
| (Note 3) | Shin-Etsu Chemical Co., Ltd.: Lauryl polyglyceryl-3 polydimethylsiloxyethyl dimethicone |
| (Note 4) | Shin-Etsu Chemical Co., Ltd.: Hydrogen dimethicone |
| (Note 5) | Shin-Etsu Chemical Co., Ltd.: KF-9909-treated coloring inorganic pigments, W: white, R: red, Y: yellow, B: black |

### (Production method)

A: Components 1 to 9 were heated to give a solution.
B: Components 12 to 15 were mixed uniformly and roll-treated.
C: The mixture obtained in step B, and components 10 and 11 were added to the hot solution obtained in step A. The resultant mixture was mixed uniformly.
D: The mixture obtained in step C was defoamed under heated conditions, then added into a container, and cooled to room temperature. An oil-based solid foundation was thus obtained.

The oil-based solid foundation obtained as described above spread lightly, was well-fitting to the skin, gave a moist finish, and formed a long-lasting firm cosmetic film with moderate luster, thus showing high usefulness.

### [Formulation Example 56: Powder foundation]

| Formulation | | % by mass |
|---|---|---|
| 1. | Composite powder obtained in Example 11 | 9.0 |
| 2. | KSP-411 (Note 1) | 1.0 |
| 3. | Polyethylene | 1.5 |
| 4. | Barium sulfate | 5.0 |
| 5. | KF-9909 (Note 2) treated mica | 40.0 |
| 6. | KF-9909 (Note 2) treated talc | balance |
| 7. | KTP-09W, R, Y, B (Note 3) | 15.0 |
| 8. | Mineral oil | 2.0 |
| 9. | Squalane | 2.0 |
| 10. | KF-4418 (Note 4) | 4.0 |
| 11. | Dimethicone (50 cs) | 1.0 |
| Total | | 100.0 |

| | |
|---|---|
| (Note 1) | Shin-Etsu Chemical Co., Ltd.: Polysilicone-1 crosspolymer |
| (Note 2) | Shin-Etsu Chemical Co., Ltd.: Triethoxysilylethyl polydimethylsiloxyethylhexyl dimethicone |
| (Note 3) | Shin-Etsu Chemical Co., Ltd.: KF-9909-treated coloring inorganic pigments, W: white, R: red, Y: yellow, B: black |
| (Note 4) | Shin-Etsu Chemical Co., Ltd.: Caprylyl methicone |

### (Production method)

A: Components 1 to 7 were mixed uniformly using a Henschel mixer.
B: Components 6 to 11 were mixed uniformly.
C: The mixture obtained in step B was added to the mixture obtained in step A. The resultant mixture was mixed uniformly using a Henschel mixer.
D: The mixture obtained in step C was passed through a sieve and was compacted in a metal dish using a mold to give a powder foundation.

The powder foundation obtained as described above showed full smoothness on application, spread lightly without stickiness, had excellent adhesion, was well-fitting, and gave an exudate-free and long-lasting finish with moderate luster, thus showing high usefulness.

### [Formulation Example 57: Loose powder]

| Formulation | | % by mass |
|---|---|---|
| 1. | Composite powder obtained in Example 7 | 20.0 |
| 2. | KSP-100 (Note 1) | 10.0 |
| 3. | KSP-300 (Note 2) | 5.0 |
| 4. | Lauroyl lysine | 5.0 |
| 5. | Boron nitride | 3.0 |
| 6. | Pearl pigment | 3.0 |
| 7. | KF-9901 (Note 3) treated synthetic fluorphlogopite | 15.0 |
| 8. | KF-9901 (Note 3) treated talc | balance |
| 9. | Disodium N-stearoyl-L-glutamate-treated inorganic coloring pigment | 4.0 |
| 10. | Isononyl isononanoate | 2.0 |
| 11. | Ethylhexylglycerin | 0.5 |
| Total | | 100.0 |

| | |
|---|---|
| (Note 1) | Shin-Etsu Chemical Co., Ltd.: (Vinyl dimethicone/methicone silsesquioxane) crosspolymer |
| (Note 2) | Shin-Etsu Chemical Co., Ltd.: (Diphenyl dimethicone/vinyl diphenyl dimethicone/silsesquioxane) crosspolymer |
| (Note 3) | Shin-Etsu Chemical Co., Ltd.: Hydrogen dimethicone |

### (Production method)

A: Components 1 to 9 were mixed uniformly using a Henschel mixer.
B: Components 10 and 11 were mixed to give a uniform solution.
C: The solution obtained in step B was added to the mixture obtained in step A. The resultant mixture was mixed uniformly using a Henschel mixer.
D: The mixture obtained in step C was passed through a sieve and then added into a container. A loose powder was thus obtained.

The loose powder obtained as described above showed full smoothness on application, gave a finish with moderate luster, and was long-lasting, thus showing high usefulness.

### [Formulation Example 58: Oil-based cheek color]

| Formulation | | % by mass |
|---|---|---|
| 1. | KSG-16 (Note 1) | 28.0 |
| 2. | Cyclopentasiloxane | balance |
| 3. | Neopentyl glycol dicaprylate | 9.0 |
| 4. | Inulin stearate | 10.0 |
| 5. | Antioxidant | q.s. |
| 6. | Composite powder obtained in Example 4 | 12.0 |
| 7. | Red #202 | 0.2 |
| 8. | KTP-09W, R, Y, B (Note 2) | 5.0 |
| 9. | KF-9909-treated pearl pigment (Note 3) | 5.0 |
| 10. | KF-9909-treated mica (Note 3) | 11.5 |
| Total | | 100.0 |

| | |
|---|---|
| (Note 1) | Shin-Etsu Chemical Co., Ltd.: Mixture of 70-80% by mass dimethicone + 20-30% by mass (dimethicone/vinyl dimethicone) crosspolymer |
| (Note 2) | Shin-Etsu Chemical Co., Ltd.: KF-9909-treated coloring inorganic pigments, W: white, R: red, Y: yellow, B: black |
| (Note 3) | Shin-Etsu Chemical Co., Ltd.: Triethoxysilylethyl polydimethylsiloxyethylhexyl dimethicone |

### (Production method)

A: Components 1 to 5 were mixed, heated to 80°C, and mixed uniformly.
B: Components 6 to 10 were mixed uniformly using a Henschel mixer.
C: The mixture obtained in step B was added to the mixture obtained in step A. The resultant mixture was mixed uniformly at 80°C.
D: The mixture obtained in step C was defoamed under heated conditions, then added into a container, and cooled to room temperature to give an oil-based cheek color.

The oil-based cheek color obtained as described above was sponge-like, was easy to pick up, spread lightly, and showed a non-oily and non-powdery feeling on use. Furthermore, the oil-based cheek color was long-lasting with good water resistance, water repellency, and sweat resistance, was resistant to coming off, and was free from changes due to temperature or aging, thus being shown to have excellent stability.

### [Formulation Example 59: Powder cheek color]

| | | |
|---|---|---|
| Formulation | | % by mass |
| 1. | Composite powder obtained in Example 13 | 7.0 |
| 2. | KSP-441 (Note 1) | 2.0 |
| 3. | Zinc laurate | 1.5 |
| 4. | Isostearyl sebacate-treated mica | 10.0 |
| 5. | Isostearyl sebacate-treated talc | balance |
| 6. | Dimethicone-treated pearl pigment | 3.0 |
| 7. | Red #202 | 0.3 |
| 8. | Yellow #4 | 0.5 |
| 9. | Isostearyl sebacate-treated inorganic coloring pigment | 3.5 |
| 10. | Triethylhexanoin | 2.0 |
| 11. | Diisostearyl malate | 1.5 |
| 12. | KF-6038 (Note 2) | 1.0 |
| 13. | Dimethicone (20 cs) | 3.0 |
| Total | | 100.0 |

| | |
|---|---|
| (Note 1) | Shin-Etsu Chemical Co., Ltd.: Polysilicone-22 |
| (Note 2) | Shin-Etsu Chemical Co., Ltd.: Lauryl PEG-9 polydimethylsiloxyethyl dimethicone |

### (Production method)

A: Components 1 to 9 were mixed uniformly using a Henschel mixer.
B: Components 10 to 13 were mixed uniformly.
C: The mixture obtained in step B was added to the mixture obtained in step A. The resultant mixture was mixed uniformly using a Henschel mixer.
D: The mixture obtained in step C was passed through a sieve and was compacted in a metal dish using a mold to give a powder cheek color.

The powder cheek color obtained as described above showed full smoothness on application, spread lightly without stickiness, had excellent adhesion, was well-fitting, and gave an exudate-free and long-lasting finish with moderate luster, thus showing high usefulness.

### [Formulation Example 60: Water-in-oil cream eyeshadow]

| Formulation | | % by mass |
|---|---|---|
| 1. | Cyclopentasiloxane | 15.0 |
| 2. | Dimethicone (6 cs) | 10.0 |
| 3. | KF-6028 (Note 1) | 2.0 |
| 4. | Pentaerythrityl tetraethylhexanoate | 5.0 |
| 5. | Composite powder obtained in Example 11 | 16.0 |
| 6. | Aluminum dimyristate-treated inorganic coloring pigment | 4.5 |
| 7. | Sodium chloride | 2.0 |
| 8. | PG | 8.0 |
| 9. | Preservative | q.s. |
| 10. | Fragrance | q.s. |
| 11. | Water | balance |
| Total | | 100.0 |

| | | |
|---|---|---|
| (Note 1) Shin-Etsu Chemical Co., Ltd.: PEG-9 polydimethylsiloxyethyl dimethicone | | |

### (Production method)

A: Components 1 to 4 were mixed uniformly.
B: Components 5 and 6 were mixed uniformly using a Henschel mixer.
C: Components 7 to 9 and 11 were mixed to give a uniform solution.
D: The solution obtained in step C was added to the mixture obtained in step A. The resultant mixture was mixed uniformly.
E: Component 10 was added to the mixture obtained in step D. The resultant mixture was mixed uniformly.
F: The mixture obtained in step E was defoamed and then added into a container. A water-in-oil cream eyeshadow was thus obtained.

The water-in-oil cream eyeshadow obtained as described above spread lightly and showed a non-oily and non-powdery feeling on use. Furthermore, the water-in-oil cream eyeshadow was long-lasting with good water resistance, water repellency, and sweat resistance, was resistant to coming off, and was free from changes due to temperature or aging, thus being shown to have excellent stability.

### [Formulation Example 61: Oil-based eye color]

| Formulation | | % by mass |
|---|---|---|
| 1. | Isotridecyl isononanoate | balance |
| 2. | Squalane | 20.0 |
| 3. | Dextrin palmitate | 10.0 |
| 4. | KSG-16 (Note 1) | 12.0 |
| 5. | Composite powder obtained in Example 12 | 6.0 |
| 6. | Barium sulfate | 5.0 |
| 7. | (PET/aluminum) laminate | 4.5 |
| 8. | Dimethicone-treated pearl pigment | 13.5 |
| 9. | Antioxidant | q.s. |
| 10. | Titanium oxide-coated (calcium/aluminum) borosilicate | 1.5 |
| 11. | Iron oxide-coated (calcium/aluminum) borosilicate | 7.5 |
| Total | | 100.0 |

| | | |
|---|---|---|
| (Note 1) Shin-Etsu Chemical Co., Ltd.: Mixture of 70-80% by mass dimethicone + 20-30% by mass (dimethicone/vinyl dimethicone) crosspolymer | | |

### (Production method)

A: Components 1 to 4 were mixed, heated to 90°C, and mixed uniformly.
B: Components 5 to 11 were added to the mixture obtained in step A. The resultant mixture was mixed uniformly at 90°C.
C: The mixture obtained in step B was defoamed under heated conditions, then added into a container, and cooled to room temperature to give an oil-based eye color.

The oil-based eye color obtained as described above was jelly-like, was easy to pick up, spread lightly, and showed a non-oily and non-powdery feeling on use. Furthermore, the oil-based eye color was long-lasting with good water resistance, water repellency, and sweat resistance, was resistant to coming off, and was free from changes due to temperature or aging, thus being shown to have excellent stability.

### [Formulation Example 62: Powder eyeshadow]

| Formulation | | % by mass |
|---|---|---|
| 1. | Composite powder obtained in Example 6 | 8.0 |
| 2. | Zinc myristate | 2.0 |
| 3. | Boron nitride | 3.0 |
| 4. | Barium sulfate | 5.0 |
| 5. | KF-9901 (Note 1) treated synthetic fluorphlogopite | 15.0 |
| 6. | KF-9901 (Note 1) treated mica | balance |
| 7. | KF-9901 (Note 1) treated pearl pigment | 10.0 |
| 8. | Red #201 | 0.2 |
| 9. | Yellow #4 | 0.3 |
| 10. | Lauroyl lysine-treated inorganic coloring pigment | 6.0 |
| 11. | Isotridecyl isononanoate | 2.0 |
| 12. | KP-561P (Note 2) | 1.0 |
| 13. | KF-56A (Note 3) | 4.0 |
| Total | | 100.0 |

| | |
|---|---|
| (Note 1) | Shin-Etsu Chemical Co., Ltd.: Hydrogen dimethicone |
| (Note 2) | Shin-Etsu Chemical Co., Ltd.: (Acrylates/stearyl acrylate/dimethicone methacrylate) copolymer |
| (Note 3) | Shin-Etsu Chemical Co., Ltd.: Diphenylsiloxyphenyl trimethicone |

### (Production method)

A: Components 1 to 10 were mixed uniformly using a Henschel mixer.
B: Components 11 to 13 were mixed uniformly.
C: The mixture obtained in step B was added to the mixture obtained in step A. The resultant mixture was mixed uniformly using a Henschel mixer.
D: The mixture obtained in step C was passed through a sieve and was compacted in a metal dish using a mold to give a powder eyeshadow.

The powder eyeshadow obtained as described above showed full smoothness on application, spread lightly without stickiness, had excellent adhesion, was well-fitting, and gave an exudate-free and long-lasting finish with moderate luster, thus showing high usefulness.

### [Formulation Example 63: Stick-shaped lip cosmetic]

| Formulation | | % by mass |
|---|---|---|
| 1. | Candelilla wax | 8.0 |
| 2. | Polyethylene | 8.0 |
| 3. | KP-561P (Note 1) | 12.0 |
| 4. | KF-54HV (Note 2) | 3.0 |
| 5. | Isotridecyl isononanoate | 15.5 |
| 6. | Glyceryl isostearate | 16.0 |
| 7. | Polyglyceryl-2 triisostearate | balance |
| 8. | AES-3083 (Note 3) treated organic coloring pigment | 2.0 |
| 9. | AES-3083 (Note 3) treated inorganic coloring pigment | 4.0 |
| 10. | Composite powder obtained in Example 2 | 6.0 |
| 11. | Fragrance | 0.1 |
| Total | | 100.0 |

| | |
|---|---|
| (Note 1) | Shin-Etsu Chemical Co., Ltd.: (Acrylates/stearyl acrylate/dimethicone methacrylate) copolymer |
| (Note 2) | Shin-Etsu Chemical Co., Ltd.: Diphenyl dimethicone |
| (Note 3) | Shin-Etsu Chemical Co., Ltd.: Triethoxycaprylylsilane |

### (Production method)

A: Components 1 to 6 and a portion of component 7 were mixed. The mixture was heated to 90°C and mixed uniformly.
B: Components 8 and 9, and the remainder of component 7 were mixed and roll-treated.
C: The mixture obtained in step B was added to the mixture obtained in step A. The resultant mixture was mixed uniformly at 90°C.
D: Components 10 and 11 were added to the mixture obtained in step C. The resultant mixture was mixed uniformly at 90°C.
E: The mixture obtained in step D was defoamed under heated conditions, then added into a container, and cooled to room temperature to give a stick-shaped lip cosmetic.

The stick-shaped lip cosmetic obtained as described above spread lightly, was not oily or powdery, was long-lasting with good water resistance and water repellency, and was excellent in stability.

### [Formulation Example 64: Water-in-oil cream lip cosmetic]

| Formulation | | % by mass |
|---|---|---|
| 1. | KSG-43 (Note 1) | 8.0 |
| 2. | KF-6105 (Note 2) | 2.0 |
| 4. | Dextrin (palmitate/ethylhexanoate) | 9.0 |
| 5. | X-21-5249 (Note 3) | 5.0 |
| 6. | Composite powder obtained in Example 10 | 8.0 |
| 7. | Cyclopentasiloxane | balance |
| 8. | Triethylhexanoin | 3.0 |
| 9. | KF-6115 (Note 4) | 0.3 |
| 10. | Red #202 | 0.3 |
| 11. | Yellow #4 | 0.5 |
| 12. | BG | 5.0 |
| 13. | Water | 10.0 |
| Total | | 100.0 |

| | |
|---|---|
| (Note 1) | Shin-Etsu Chemical Co., Ltd.: Mixture of 65-75% by mass triethylhexanoin + 25-35% by mass (vinyl dimethicone/lauryl dimethicone) crosspolymer |
| (Note 2) | Shin-Etsu Chemical Co., Ltd.: Lauryl polyglyceryl-3 polydimethylsiloxyethyl dimethicone |
| (Note 3) | Shin-Etsu Chemical Co., Ltd.: Solution of 50% by mass cyclopentasiloxane + 50% by mass trimethylsiloxysilicate |
| (Note 4) | Shin-Etsu Chemical Co., Ltd.: Lauryl polyglyceryl-3 polydimethylsiloxyethyl dimethicone |

### (Production method)

A: Components 1 to 7 were mixed. The mixture was heated to 90°C and mixed uniformly.
B: Components 8 to 11 were mixed and roll-treated.
C: Components 12 and 13 were mixed to give a uniform solution.
D: The mixture obtained in step B was added to the mixture obtained in step A. The resultant mixture was mixed uniformly at 90°C.
E: The solution obtained in step C was added to the mixture obtained in step D. The resultant mixture was mixed uniformly at 90°C.
F: The mixture obtained in step E was cooled to room temperature, defoamed, and then added into a container. A water-in-oil cream lip cosmetic was thus obtained.

The water-in-oil cream lip cosmetic obtained as described above showed excellent characteristics, specifically, had excellent spreadability and smoothness on application, was free of stickiness, had excellent adhesion, was well-fitting, gave a beautiful finish with reduced unnatural luster, was long-lasting without exudate, color transfer, or color fading, and did not produce dryness or roughness on the lips.

### [Formulation Example 65: Lip gloss]

| Formulation | | % by mass |
|---|---|---|
| 1. | Glyceryl tri(behenate/isostearate/eicosanedioate) | 8.0 |
| 2. | Hydrogenated polyisobutene | 30.0 |
| 3. | KSG-44 (Note 1) | 6.0 |
| 4. | KP-545L (Note 2) | 3.0 |
| 5. | Isotridecyl isononanoate | balance |
| 6. | Composite powder obtained in Example 11 | 5.0 |
| 7. | Ethylhexyl palmitate | 3.0 |
| 8. | KF-6015 (Note 3) | 0.5 |
| 9. | Red #202 | 0.1 |
| 10. | Yellow #4 | 0.2 |
| 11. | KF-99P (Note 4) treated inorganic coloring pigment | 1.0 |
| 12. | Pearl pigment | q.s. |
| Total | | 100.0 |

| | |
|---|---|
| (Note 1) | Shin-Etsu Chemical Co., Ltd.: Mixture of 65-75% by mass squalane + 25-35% by mass (vinyl dimethicone/lauryl dimethicone) crosspolymer |
| (Note 2) | Shin-Etsu Chemical Co., Ltd.: Solution of 60% by mass dimethicone (2 cs) + 40% by mass (acrylates/dimethicone) copolymer |
| (Note 3) | Shin-Etsu Chemical Co., Ltd.: PEG-3 dimethicone |
| (Note 4) | Shin-Etsu Chemical Co., Ltd.: Methicone |

### (Production method)

A: Components 1 to 6 were mixed uniformly.
B: Components 7 to 11 were mixed uniformly and roll-treated.
C: The mixture obtained in step B was added to the mixture obtained in step A. The resultant mixture was mixed uniformly.
D: Component 12 was added to the mixture obtained in step C. The resultant mixture was mixed uniformly.
E: The mixture obtained in step D was defoamed and then added into a container. A lip gloss was thus obtained.

The lip gloss obtained as described above showed excellent characteristics, specifically, had excellent spreadability and smoothness on application, was free of stickiness, had excellent adhesion, was well-fitting, gave a beautiful finish with reduced unnatural luster, was long-lasting without exudate, color transfer, or color fading, and did not produce dryness or roughness on the lips.

### [Formulation Example 66: Oil-based mascara]

| Formulation | | % by mass |
|---|---|---|
| 1. | Paraffin | 20.0 |
| 2. | Microcrystalline wax | 8.0 |
| 3. | Polyethylene | 3.0 |
| 4. | Inulin stearate | 1.0 |
| 5. | Disteardimonium hectorite | 2.0 |
| 6. | KF-6028 (Note 1) | 1.0 |
| 7. | NBN-30-ID (Note 2) | 3.0 |
| 8. | Hydrogenated polyisobutene | balance |
| 9. | Composite powder obtained in Example 3 | 2.0 |
| 10. | Neopentyl glycol dicaprylate | 5.0 |
| 11. | KF-6115 (Note 3) | 1.0 |
| 12. | KTP-09W, B (Note 4) | 6.5 |
| Total | | 100.0 |

| | |
|---|---|
| (Note 1) | Shin-Etsu Chemical Co., Ltd.: PEG-9 polydimethylsiloxyethyl dimethicone |
| (Note 2) | Shin-Etsu Chemical Co., Ltd.: Solution of 70% by mass isododecane + 30% by mass norbornene/tris(trimethylsiloxy)silylnorbornene) copolymer |
| (Note 3) | Shin-Etsu Chemical Co., Ltd.: Lauryl polyglyceryl-3 polydimethylsiloxyethyl dimethicone |
| (Note 4) | Shin-Etsu Chemical Co., Ltd.: KF-9909-treated coloring inorganic pigments, W: white, B: black |

### (Production method)

A: Components 1 to 8 were heated to give a solution.
B: Components 10 to 13 were mixed uniformly and roll-treated.
C: The mixture obtained in step B and component 9 were added to the hot solution obtained in step A. The resultant mixture was mixed uniformly.
D: The mixture obtained in step C was defoamed, cooled to room temperature, and added into a container. An oil-based mascara was thus obtained.

The oil-based mascara obtained as described above showed full smoothness on application, spread lightly without stickiness, had excellent adhesion, was well-fitting, and gave an exudate-free and long-lasting finish with moderate luster, thus showing high usefulness.

### [Formulation Example 67: Water-in-oil eyeliner]

| Formulation | | % by mass |
|---|---|---|
| 1. | Cyclopentasiloxane | balance |
| 2. | KF-6017P (Note 1) | 3.0 |
| 3. | KF-7312J (Note 2) | 15.0 |
| 4. | Disteardimonium hectorite | 2.0 |
| 5. | KF-9901 (Note 3) treated inorganic coloring pigment | 8.0 |
| 6. | Composite powder obtained in Example 11 | 12.0 |
| 7. | BG | 5.0 |
| 8. | Preservative | q.s. |
| 9. | Water | 15.0 |
| Total | | 100.0 |

| | |
|---|---|
| (Note 1) | Shin-Etsu Chemical Co., Ltd.: PEG-10 dimethicone |
| (Note 2) | Shin-Etsu Chemical Co., Ltd.: Solution of 50% by mass cyclopentasiloxane + 50% by mass trimethylsiloxysilicate |
| (Note 3) | Shin-Etsu Chemical Co., Ltd.: Hydrogen dimethicone |

### (Production method)

A: Components 1 to 4 were mixed, and components 5 and 6 were added. The resultant mixture was mixed uniformly.
B: Components 7 to 9 were mixed to give a uniform solution.
C: The solution obtained in step B was added to the mixture obtained in step A. The resultant mixture was mixed uniformly.
D: The mixture obtained in step C was defoamed and then added into a container. A water-in-oil eyeliner was thus obtained.

The water-in-oil eyeliner obtained as described above spread lightly, was easy to apply, gave a fresh, non-oily, and non-sticky feeling on use, and was free from changes due to temperature or aging, thus being shown to have excellent usability and stability. Furthermore, the water-in-oil eyeliner had excellent water resistance and sweat resistance, and was very long-lasting.

### [Formulation Example 68: Water-in-oil eyeliner]

| Formulation | | % by mass |
|---|---|---|
| 1. | Cyclopentasiloxane | balance |
| 2. | Dimethicone (6 cs) | 5.0 |
| 3. | Jojoba oil | 2.0 |
| 4. | KF-6017 (Note 1) | 1.0 |
| 5. | KF-6038 (Note 2) | 1.0 |
| 6. | KP-545 (Note 3) | 15.0 |
| 7. | Composite powder obtained in Example 9 | 10.0 |
| 8. | KTP-09B (Note 4) | 18.0 |
| 9. | Ethanol | 5.0 |
| 10. | Preservative | q.s. |
| 11. | Water | 35.0 |
| Total | | 100.0 |

| | |
|---|---|
| (Note 1) | Shin-Etsu Chemical Co., Ltd.: PEG-10 dimethicone |
| (Note 2) | Shin-Etsu Chemical Co., Ltd.: Lauryl PEG-9 polydimethylsiloxyethyl dimethicone |
| (Note 3) | Shin-Etsu Chemical Co., Ltd.: Solution of 70% by mass cyclopentasiloxane + 30% (acrylates/dimethicone) copolymer |
| (Note 4) | Shin-Etsu Chemical Co., Ltd.: KF-9909-treated coloring inorganic pigment, B: black |

### (Production method)

A: Components 1 to 6 were mixed, and components 7 and 8 were added. The resultant mixture was mixed uniformly.
B: Components 9 to 11 were mixed to give a uniform solution.
C: The solution obtained in step B was added to the mixture obtained in step A. The resultant mixture was mixed uniformly.
D: The mixture obtained in step C was defoamed and then added into a container. A water-in-oil eyeliner was thus obtained.

The water-in-oil eyeliner obtained as described above spread lightly, was not oily or powdery, was long-lasting with good water resistance, water repellency, and sweat resistance, and was resistant to coming off. Furthermore, the water-in-oil eyeliner was free from changes due to temperature or aging, thus being shown to have excellent stability.

### [Formulation Example 69: Water-in-oil eyeliner]

| | Formulation | % by mass |
|---|---|---|
| 1. | Cyclopentasiloxane | balance |
| 2. | Dimethicone (6 cs) | 2.0 |
| 3. | AES-3083 (Note 1) treated inorganic coloring pigment | 20.0 |
| 4. | Composite powder obtained in Example 14 | 4.0 |
| 5. | X-21-5595 (Note 2) | 10.0 |
| 6. | Jojoba oil | 2.0 |
| 7. | Bentonite | 3.0 |
| 8. | KF-6017 (Note 3) | 2.0 |
| 9. | Antioxidant | q.s. |
| 10. | Ethanol | 3.0 |
| 11. | Pentylene glycol | 5.0 |
| 12. | Preservative | q.s. |
| 13. | Water | 20.0 |
| | Total | 100.0 |

| | |
|---|---|
| (Note 1) | Shin-Etsu Chemical Co., Ltd.: Triethoxycaprylylsilane |
| (Note 2) | Shin-Etsu Chemical Co., Ltd.: Solution of 40% by mass isododecane + 60% by mass trimethylsiloxysilicate |
| (Note 3) | Shin-Etsu Chemical Co., Ltd.: PEG-10 dimethicone |

### (Production method)

A: Components 1, 2, and 5 to 9 were mixed, and components 3 and 4 were added. The resultant mixture was mixed uniformly.
B: Components 10 to 13 were mixed.
C: The mixture obtained in step B was added to the mixture obtained in step A. The resultant mixture was mixed uniformly.
D: The mixture obtained in step C was defoamed and then added into a container. A water-in-oil eyeliner was thus obtained.

The water-in-oil eyeliner obtained as described above spread lightly, was easy to apply, gave a fresh, non-oily, and non-sticky feeling on use, was excellent in water resistance and sweat resistance, and was very long-lasting. Furthermore, the water-in-oil eyeliner was free from changes due to temperature or aging.

### [Formulation Example 70: Water-in-oil antiperspirant cream]

| | Formulation | % by mass |
|---|---|---|
| 1. | KSG-210 (Note 1) | 7.0 |
| 2. | Cyclopentasiloxane | 10.0 |
| 3. | Neopentyl glycol diethylhexanoate | 7.0 |
| 4. | Composite powder obtained in Example 6 | 10.0 |
| 5. | KSP-102 (Note 2) | 5.0 |
| 6. | DPG | 5.0 |
| 7. | Sodium citrate | 0.2 |
| 8. | Aluminum/zirconium trichlorohydrex glycine | 18.0 |
| 9. | Fragrance | q.s. |
| 10. | Water | balance |
| | Total | 100.0 |

| | |
|---|---|
| (Note 1) | Shin-Etsu Chemical Co., Ltd.: Mixture of 70-80% by mass dimethicone + 20-30% by mass (dimethicone/(PEG-10/15)) crosspolymer |
| (Note 2) | Shin-Etsu Chemical Co., Ltd.: (Vinyl dimethicone/methicone silsesquioxane) crosspolymer |

### (Production method)

A: Components 1 to 5 were mixed uniformly.
B: Components 6 to 10 were mixed to give a uniform solution.
C: The solution obtained in step B was added to the mixture obtained in step A. The resultant mixture was mixed uniformly.
D: The mixture obtained in step C was defoamed and then added into a container. A water-in-oil antiperspirant cream was thus obtained.

The water-in-oil antiperspirant cream obtained as described above spread lightly without stickiness or oiliness, and was free from changes due to temperature or aging, thus being shown to have excellent usability and stability.

### [Formulation Example 71: Roll-on type antiperspirant cosmetic]

| | Formulation | % by mass |
|---|---|---|
| 1. | KSG-210 (Note 1) | 20.0 |
| 2. | Dimethicone (6 cs) | 10.0 |
| 3. | KSG-15 (Note 2) | 15.0 |
| 4. | Cyclopentasiloxane | balance |
| 5. | Aluminum/zirconium trichlorohydrex glycine | 20.0 |
| 6. | Composite powder obtained in Example 6 | 20.0 |
| 7. | Fragrance | q.s. |
| | Total | 100.0 |

### (Production method)

A: Components 1 to 4 were mixed uniformly.
B: Components 5 to 7 were added to the mixture obtained in step A. The resultant mixture was mixed uniformly.
C: The mixture obtained in step B was defoamed and then added into a container. A roll-on type antiperspirant cosmetic was thus obtained.

The roll-on type antiperspirant cosmetic obtained as described above spread lightly without stickiness or oiliness, and was free from changes due to temperature or aging, thus being shown to have excellent usability and stability.

### [Formulation Example 72: Oil-based antiperspirant cream]

| | Formulation | % by mass |
|---|---|---|
| 1. | KF-4422 (Note 1) | 30.0 |
| 2. | KSG-15 (Note 2) | 21.5 |
| 3. | Neopentyl glycol dioctanoate | balance |
| 4. | Polyethylene | 3.0 |
| 5. | Ceresin | 6.0 |
| 6. | Antioxidant | q.s. |
| 7. | Aluminum/zirconium trichlorohydrex glycine | 19.0 |
| 8. | Composite powder obtained in Example 6 | 10.0 |
| 9. | Dimethylsilylated silica | 0.5 |
| 10. | Fragrance | q.s. |
| | Total | 100.0 |

| | |
|---|---|
| (Note 1) | Shin-Etsu Chemical Co., Ltd.: Ethyl trimethicone |
| (Note 2) | Shin-Etsu Chemical Co., Ltd.: Mixture of 90-96% by mass cyclopentasiloxane + 4-10% by mass (dimethicone/vinyl dimethicone) crosspolymer |

### (Production method)

A: Components 1 to 6 were heated and mixed uniformly.
B: Components 7 to 9 were added to the mixture obtained in step A. The resultant mixture was mixed uniformly.
C: Component 10 was added to the mixture obtained in step B. The resultant mixture was mixed uniformly.
D: The mixture obtained in step C was defoamed and then added into a container. An oil-based antiperspirant cream was thus obtained.

The oil-based antiperspirant cream obtained as described above had good spreadability and spread very smoothly, was not excessively dry or sticky, and showed long-lasting deodorizing effects.

### [Formulation Example 73: Nail enamel]

| | Formulation | % by mass |
|---|---|---|
| 1. | KP-549 (Note 1) | 44.0 |
| 2. | TMF-1.5 (Note 2) | 5.0 |
| 3. | Nitrocellulose | 3.0 |
| 4. | Camphorquinone | 0.5 |
| 5. | Acetyl tributyl citrate | 1.0 |
| 6. | Dimethyl distearyl ammonium hectorite | 0.5 |
| 7. | Butyl acetate | 30.0 |
| 8. | Ethyl acetate | 10.0 |
| 9. | Isopropyl alcohol | 5.0 |
| 10. | Composite powder obtained in Example 10 | 1.0 |
| | Total | 100.0 |

| | |
|---|---|
| (Note 1) | Shin-Etsu Chemical Co., Ltd.: Solution of 60% by mass methyl trimethicone + 40% (acrylates/dimethicone) copolymer |
| (Note 2) | Shin-Etsu Chemical Co., Ltd.: Methyl trimethicone |

### (Production method)

A: Components 7 to 9 were mixed, and components 4 to 6 were added. The mixture was mixed uniformly.
B: Components 1 to 3 were added to the mixture obtained in step A. The resultant mixture was mixed uniformly.
C: Component 10 was added to the mixture obtained in step B. The resultant mixture was mixed uniformly.
D: The mixture obtained in step C was defoamed and then added into a container. A nail enamel was thus obtained.

The nail enamel obtained as described above spread lightly, gave a visually smooth appearance, was water-resistant and oil-resistant and was long-lasting, did not cause a feeling of pressure on the nail, did not cause yellowing of the nail, and was free from changes in the cosmetic film due to temperature or aging, thus being shown to have excellent stability.

### [Formulation Example 74: Aqueous gel]

| | Formulation | % by mass |
|---|---|---|
| 1. | Composite powder obtained in Example 16 | 5.0 |
| 2. | KF-6043 (Note 1) | 0.5 |
| 3. | Ethanol | 3.0 |
| 4. | BG | 4.0 |
| 5. | Glycerin | 2.0 |
| 6. | (Ammonium acryloyldimethyltaurate/VP) copolymer | 0.2 |
| 7. | Xanthan gum | 0.2 |
| 8. | Arginine | 0.5 |
| 9. | Preservative | q.s. |
| 10. | Water | balance |
| | Total | 100.0 |

| | | |
|---|---|---|
| (Note 1) Shin-Etsu Chemical Co., Ltd.: PEG-10 dimethicone | | |

### (Production method)

A: Components 1 to 3 were mixed uniformly.
B: Components 4 to 10 were mixed uniformly.
C: The mixture obtained in step A was added to the mixture obtained in step B. The resultant mixture was mixed uniformly.
D: The mixture obtained in step C was defoamed and then added into a container. An aqueous gel was thus obtained.

The aqueous gel obtained as described above gave a highly dewy feeling on application, spread lightly without stickiness, had excellent adhesion, was well-fitting, and gave a matte finish with moderate luster.

### [Formulation Example 75: Oil-based gel]

| Formulation | | % by mass |
|---|---|---|
| 1. | Composite powder obtained in Example 17 | 10.0 |
| 2. | KSG-19 (Note 1) | 20.0 |
| 3. | KSG-15 (Note 2) | 30.0 |
| 4. | KF-56A (Note 3) | 5.0 |
| 5. | Dimethicone (1.5 cs) | balance |
| Total | | 100.0 |

| | |
|---|---|
| (Note 1) | Shin-Etsu Chemical Co., Ltd.: Mixture of 80-90% by mass dimethicone + 10-20% by mass (dimethicone/vinyl dimethicone) crosspolymer |
| (Note 2) | Shin-Etsu Chemical Co., Ltd.: Mixture of 90-96% by mass cyclopentasiloxane + 4-10% by mass (dimethicone/vinyl dimethicone) crosspolymer |
| (Note 3) | Shin-Etsu Chemical Co., Ltd.: Diphenylsiloxyphenyl trimethicone |

### (Production method)

A: Components 1 to 5 were mixed uniformly.
B: The mixture obtained in step A was defoamed and then added into a container. An oil-based gel was thus obtained.

The oil-based gel obtained as described above showed full smoothness on application, spread lightly without stickiness, had excellent adhesion, was well-fitting, and gave a matte finish with moderate luster.

### [Formulation Example 76: Oil-based mousse foundation]

| Formulation | | % by mass |
|---|---|---|
| 1. | Dimethicone (6 cs) | balance |
| 2. | KF-7312L (Note 1) | 10.0 |
| 3. | KSG-048Z (Note 2) | 30.0 |
| 4. | Squalane | 1.0 |
| 5. | Jojoba oil | 1.0 |
| 6. | KF-56A (Note 3) | 1.0 |
| 7. | Composite powder obtained in Example 16 | 18.0 |
| 8. | Methyl methacrylate crosspolymer | 1.0 |
| 9. | Nylon-12 | 1.0 |
| 10. | KTP-09W, R, Y, B (Note 4) | 10.0 |
| 11. | Stearic acid-treated titanium oxide microparticles | 10.0 |
| 12. | Antioxidant | q.s. |
| 13. | Dimethicone-treated talc | 3.0 |
| 14. | Dimethicone-treated mica | 5.0 |
| Total | | 100.0 |

| | |
|---|---|
| (Note 1) | Shin-Etsu Chemical Co., Ltd.: Solution of 50% by mass dimethicone (2 cs) + 50% trimethylsiloxysilicate |
| (Note 2) | Shin-Etsu Chemical Co., Ltd.: Mixture of 75-85% by mass dimethicone + 15-25% by mass (lauryl polydimethylsiloxyethyl dimethicone/bis-vinyl dimethicone) crosspolymer |
| (Note 3) | Shin-Etsu Chemical Co., Ltd.: Diphenylsiloxyphenyl trimethicone |
| (Note 4) | Shin-Etsu Chemical Co., Ltd.: KF-9909-treated coloring inorganic pigments, W: white, R: red, Y: yellow, B: black |

### (Production method)

A: A portion of component 1, and components 2 to 9 were mixed uniformly.
B: Components 10 to 14 were mixed with the remainder of component 1. The mixture was roll-treated.
C: The mixture obtained in step B was added to the mixture obtained in step A. The resultant mixture was mixed uniformly.
D: The mixture obtained in step C was defoamed and then added into a container. An oil-based mousse foundation was thus obtained.

The mousse foundation obtained as described above was in a souffle-like form, was easy to pick up, spread lightly, and showed a non-oily and non-powdery feeling on use. Furthermore, the mousse foundation was long-lasting with good water resistance, water repellency, and sweat resistance, was resistant to coming off, and was free from changes due to temperature or aging, thus being shown to have excellent stability.

## Claims

1. A silica-coated plate-like powder comprising a plate-like powder and a coating layer of mesoporous silica.

2. The silica-coated plate-like powder according to claim 1, wherein the mesoporous silica represents 10 to 60% by mass of the silica-coated plate-like powder taken as 100% by mass.

3. The silica-coated plate-like powder according to claim 1, wherein spherical silica is further immobilized.

4. The silica-coated plate-like powder according to claim 3, wherein the spherical silica has an average particle diameter of 100 to 500 nm.

5. The silica-coated plate-like powder according to claim 3, wherein the amount of the spherical silica is 5 to 25% by mass relative to the silica-coated plate-like powder taken as 100% by mass.

6. The silica-coated plate-like powder according to claim 1, which has a BET specific surface area of 10 to 400 m²/g.

7. The silica-coated plate-like powder according to claim 1, wherein the plate-like powder is a mineral, a metal oxide, a metal, or glass.

8. The silica-coated plate-like powder according to claim 1, wherein the plate-like powder is mica.

9. A cosmetic comprising the silica-coated plate-like powder described in claim 1.

10. The cosmetic according to claim 9, which is a skin care cosmetic.

11. The cosmetic according to claim 9, which is a make-up cosmetic.

12. A silica-coated plate-like powder coated with a layer of silica having pores, the silica-coated plate-like powder being obtained by steps comprising:
step (1): preparing an aqueous solution containing a cationic surfactant at a concentration 5 times or less a critical micelle concentration;
step (2): immersing a plate-like powder in the aqueous solution obtained in the step (1), adding a silica source that generates a silanol compound upon hydrolysis to a concentration of 10 to 500 mmol/L, and stirring the mixture at a temperature of 0 to 100°C to coat a surface of the plate-like powder with a layer of mesoporous silica; and
step (3): firing a resultant silica-coated plate-like powder coated with a mesoporous silica layer to remove the cationic surfactant.

13. A method for producing a silica-coated plate-like powder coated with a layer of silica having pores, the method comprising:
step (1): preparing an aqueous solution containing a cationic surfactant at a concentration 5 times or less a critical micelle concentration;
step (2): immersing a plate-like powder in the aqueous solution obtained in the step (1), adding a silica source that generates a silanol compound upon hydrolysis to a concentration of 10 to 500 mmol/L, and stirring the mixture at a temperature of 0 to 100°C to coat a surface of the plate-like powder with a layer of mesoporous silica; and
step (3): firing a resultant silica-coated plate-like powder coated with a mesoporous silica layer to remove the cationic surfactant.
